# EUROPÄISCHE PATENTSCHRIFT

(11) **EP 1 406 607 B1**
(45) Veröffentlichungstag und Bekanntmachung des Hinweises auf die Patenterteilung: **30.11.2005**
(21) Anmeldenummer: 02759851.5
(22) Anmeldetag: 15.07.2002
(51) Int. Cl.: A61K 31/325, A61K 31/40, A61P 31/16

(54) **VERWENDUNG EINER ANTIVIRALEN VERBINDUNGSKLASSE (DITHIOCARBAMAT-VERBINDUNGEN) ZUR HERSTELLUNG EINES MITTELS ZUR BEHANDLUNG BZW. PRÄVENTION EINER VIRUSINFEKTION IM RESPIRATORISCHEN TRAKT**
VERWENDUNG EINER ANTIVIRALEN VERBINDUNGSKLASSE (DITHIOCARBAMAT-VERBINDUNGEN) ZUR HERSTELLUNG EINES MITTELS ZUR BEHANDLUNG BZW. PRAVENTION EINER VIRUSINFEKTION IM RESPIRATORISCHEN TRAKT
UTILISATION D'UNE CLASSE DE COMPOSES ANTIVIRAUX POUR PRODUIRE UN PRODUIT DESTINE A TRAITER OU A PREVENIR UNE INFECTION VIRALE DANS LA ZONE RESPIRATOIRE

(30) Priorität: 16.07.2001 AT 11032001; 16.07.2001 AT 11022001; 17.12.2001 AT 19732001; 17.12.2001 AT 19722001
(43) Veröffentlichungstag der Anmeldung: 14.04.2004
(73) Patentinhaber: Greenhills Biotechnology Research Development Trade GmbH, 1180 Wien (AT)
(72) Erfinder: Gaudernak, Elisabeth, 1120 Wien (AT); Grassauer, Andreas, 1030 Wien (AT); Küchler, Ernst, 1190 Wien (AT); Muster, Thomas, 1190 Wien (AT); Seipelt, Joachim, 1180 Wien (AT)
(74) Vertreter: Sonn & Partner Patentanwälte
(86) Internationale Anmeldenummer: PCT/AT2002/000206
(87) Internationale Veröffentlichungsnummer: WO 2003/007935

(56) Entgegenhaltungen:
- WO-A-01/51046
- WO-A-95/03792
- WO-A-99/08665
- WO-A-99/64068
- US-A- 6 093 743
- PATENT ABSTRACTS OF JAPAN vol. 001, no. 027 (C-008), 28. März 1977 (1977-03-28) & JP 51 139632 A (KAO CORP), 2. Dezember 1976 (1976-12-02)

## Beschreibung

Die Erfindung betrifft eine Verwendung von Dithiocarbamat-Verbindungen sowie ein Desinfektionsmittel und ein Verfahren zur Desinfektion von Oberflächen, Medien bzw. Zellkulturen.

Es gibt eine Vielzahl von Viren, die zu Erkrankungen im respiratorischen Trakt von Menschen und Säugetieren führen. Obgleich diese respiratorisch pathogenen Viren strukturell unterschiedlich sein können und verschiedenen Virus-Familien zugehören, so ist allen diesen Viren gemein, dass sie in der Lage sind, in den Körpern durch den respiratorischen Trakt einzudringen, indem sie z.B. spezifische Zellen dieses Traktes befallen können, z.B. die epitheliale Zellschicht im respiratorischen Trakt, Alveolen-Zellen, Lungenzellen,.... Allen gemein sind die klassischen Symptome des grippalen Infektes, der durch lokale Inflammation und Krankheitserscheinungen im respiratorischen Trakt (wie Schnupfen, Heiserkeit, Husten, Bläschen, Halsweh) gekennzeichnet ist.

Virale Infektionen, vor allem im respiratorischen Trakt, verursachen pathologische Veränderungen in den betroffenen Zellen, insbesondere in Epithelzellen, die durch oxidativen Stress bedingt sind. Reaktive Sauerstoff-Zwischenprodukte (reactive oxygen intermediates; ROIs), die z.B. durch Leukozyten, epitheliale Lungenzellen oder Xanthin-Oxidasen produziert werden, gelten als Mediatoren dieser Virus-induzierten Zellschäden. Im Zuge dieses oxidativen Stresses kann es zur Aktivierung des Oxidantien-spezifischen Transkriptionsfaktors NFκB (nuclear factor-κB) kommen. NFκB wurde in verschiedensten Zelltypen gefunden und wurde in Zusammenhang mit der Genaktivierung für Entzündungs- und Immunantworten gebracht.

Antioxidantien können die Aktivierung von NFκB blockieren, indem die ROIs, die sonst zu dieser Aktivierung führen, abgefangen werden. Daher wurde die Verwendung von Antioxidantien vor allem bei der Behandlung von Infektionen mit latenten Viren vorgeschlagen; es zeigte sich aber, dass eine effektive Behandlung dieser latenten Infektionen mit einzelnen Antioxidantien alleine nicht möglich ist, sondern - wenn überhaupt - nur durch eine kombinierte Therapie mittels einer Mischung aus verschiedenen (d.h. verschieden wirkenden) Antioxidantien und anderen virushemmenden Mitteln (US 5,686,436). Eine Inhibierung der viralen Replikation oder gar der Virusinfektion durch Antioxidantien konnte bislang aber nicht erreicht werden, insbesondere nicht für Infektionen mit Influenza- oder Picornaviren (Knobil et al. Am.J.Physiol. 274(1) (1998) (134-142).

Auf der anderen Seite sind auch die verschiedenen für die Bekämpfung von virusinfektionen vorgeschlagenen Antioxidantien in Bezug auf ihre antioxidative Aktivität sehr verschieden. So dienen L-Ascorbinsäure und Vitamin E zum Schutz von Glutathion; die Vitamine K, A und E wirken als Antagonisten von Peroxynitrit und anderen starken Oxidantien im Körper. Anti-inflammatorische Steroide, Nicht-Glukokortikoide Lazaroide, Dithiocarbamate oder N-Acetyl-L-Cystein sind als Inhibitoren der NFκB-Aktivierung beschrieben worden.

Viren können je nach ihrem Träger der genetischen Informationen eingeteilt werden in DNA und RNA-Viren, wobei die Nukleinsäure einzelsträngig oder doppelsträngig vorliegt und von einer Proteinhülle umgeben ist.

Die einzelsträngige RNA dieser RNA-Viren liegt dabei entweder als Plus-Strang (mRNA) oder Minus-Strang vor. Weiters kann diese genetische Information des Virus auch in mehreren Stücken vorhanden sein, wie zum Beispiel im Falle des Influenzavirus.

Die zu den Picornaviren zählenden humanen Rhinoviren (HRV) sind die Hauptursache für die weltweit vorkommende Erkältung. Das häufige Auftreten von HRV, das Risiko von schwerwiegenden Sekundärinfektionen und der wirtschaftliche Einfluss hinsichtlich medizinischer Kosten, Arztbesuche, Krankenstände von Angestellten machen HRV zu wichtigen, ernstzunehmenden Krankheitserregern. Trotz des häufigen Vorkommens gibt es zur Zeit keine sichere Bekämpfung dieser Viruserkrankung, abgesehen von einer Behandlung der Symptome. Auf der anderen Seite sind die Folgen z.B. einer Rhinovirus-Infektion nicht so schwerwiegend oder gar lebensbedrohend, dass die Einnahme von Arzneimitteln, die ein hohes Nebenwirkungsrisiko haben, in Kauf genommen werden darf. Mittel, die gegen derartige Viren eingesetzt werden sollen, dürfen daher nur geringe bzw. keine Nebenwirkungen aufweisen. Zur Gruppe der tierischen Picornaviren zählt das Equine Rhinitis A-Virus (ERAV), das ebenso wie das Maul- und Klauenseuche(MKS)-Virus zum Genus der Aphthoviren gehört.

Weitere wichtige Virus-Familien, die für Erkrankungen im respiratorischen Trakt verantwortlich sind, sind die Orthomyxo- und Paramyxoviridae mit dem humanen Influenza-Virus als wichtigsten Vertreter.

Das Auftreten von neuen pandemischen Influenza-Stämmen ist üblicherweise auf neue Subtypen, die ein neues Hämagglutinin bzw. das Neuraminidase-Gen enthalten, zurückzuführen. Diese neuen Viren unterscheiden sich von bisher zirkulierenden Influenza-Viren immunologisch. Um infektiös zu sein, benötigen Influenza A- und B-Viren 8 RNA-Segmente, Influenza C-Viren hingegen nur 7. Influenza A-, B- und C-Viren können in vivo homotypische Reassortanten bilden, aber nicht zwischen den Typen. Theoretisch könnte aus den jeweils 8 Segmenten von zwei Influenza A-Viren 256 Reassortanten entstehen. Diese zufällige Segregation findet aber nicht statt, weil auf der Protein-Ebene einige Proteine ihren stammspezifischen Partner brauchen. Dies wurde im speziellen klar gezeigt für das Avian Influenza-Virus A/chicken/Germany/34 (H7N1) FPV Rostock HA, welches vom Segment 4 codiert wird, das nur spezifisch mit seinem stammspezifischen M2 Protein, welches vom Segment 7 codiert wird, ein funktionelles Virus bilden kann. (Grambas S, Hay AJ. Maturation of Influenza A virus hemagglutinin-estimates of the pH encountered during transport and its regulation by the M2 protein. Virology 1992; 190:11-18) (Grambas S, Bennet HS, Hay AJ. Influence of amantadine resistance mutations on the pH regulatory function of the M2 protein of influenza A viruses. Virology 1992; 191:541-549). Eine der Haupt-Strategien gegen die jährliche Influenza-Virusinfektion in der Bevölkerung stellt die Influenza-Impfung dar. Dennoch bleibt trotz breiten Impfprogrammen Influenza ein Grund für die Morbidität und Mortalität auf der Welt und ein Hauptgrund für Erkrankung und Tod bei Patienten mit Immunschwächen bzw. bei älteren Personen. Die antivirale Aktivität von Amantadin und Rimantadin verringert die Dauer der Symptome von klinischer Influenza, aber wichtige Nebeneffekte und das Auftreten von resistenten Mutanten wurden beschrieben (FIELDS et al., Virology, 3. Edition (1995) Lippincott-Raven Publ., Philadelphia, Vol.1, p. 434-436). Zur zeit ist eine neue Gruppe von antiviralen Agentien am Markt, die die Influenzavirus-Neuraminidase inhibiert. Zanamivir und Oseltamivir sind beispielsweise Inhibitoren der Influenza A- und B-Virus-Neuraminidase; diese Medikamente verringern jedoch lediglich die Dauer der Symptome.

In der US 5 686 436 wird ein Verfahren zur Unterdrückung der Vermehrung von Retroviren und latenten Viren in Menschen und Tieren, wie beispielsweise HIV beschrieben, wobei ein Medikament umfassend unter anderem Antioxidantien und NFκB-Induktionsinhibitoren verabreicht wird. Demgegenüber muss bei einer effektiven Bekämpfung von Infektionen mit respiratorischen Viren bereits in der akuten Infektionsphase das Virus wirksam bekämpft werden; eine Behandlung, die nur auf Ebene der latenten Viren eingesetzt werden kann, ist ungeeignet zur Verhinderung oder Behandlung von viralen Akut-Infektionen im respiratorischen Trakt.

Es besteht somit das Bedürfnis nach einem hoch effektiven Wirkstoff gegen Virus-Infektionen im respiratorischen Trakt, insbesondere im Menschen, der keine oder nur geringe Nebenwirkungen auslöst, kostengünstig und in großen Mengen herstellbar ist.

Die Aufgabe der vorliegenden Erfindung wird gelöst durch die Verwendung von Dithiocarbamat-Verbindungen der Strukturformel R₁R₂NCS₂H, worin R₁ und R₂ unabhängig voneinander ein gerades oder verzweigtes C₁-C₄-Alkyl darstellen oder mit dem Stickstoffatom einen aliphatischen Ring mit 4 bis 6 C-Atomen bilden, wobei R₁, R₂ oder der aliphatische Ring gegebenenfalls mit einem oder mehreren Substituenten ausgewählt aus OH, NO₂, NH₂, COOH, SH, F, Cl, Br, I, Methyl oder Ethyl substituiert ist, und oxidierte Formen dieser Verbindungen, insbesondere Dimere hievon, sowie pharmazeutisch akzeptable Salze hievon, zur Herstellung eines Mittels zur Behandlung bzw. Verhinderung einer Infektion durch RNA-Viren, die den respiratorischen Trakt befallen und dort eine Erkrankung auslösen. Unter "respiratorischem Trakt" werden erfindungsgemäß sämtliche Organe und Bereiche ausgehend von den Körperöffnungen (Nase, Mund, Augen (inklusive Tränenkanal) und Ohren) zu den Lungenbläschen verstanden. Die pharmazeutisch akzeptablen Salze sind dabei insbesonders Na, K, Ca, Mg, NH₄ und Zn. Es wurde nun erstmals überraschenderweise gefunden, dass die erfindungsgemäßen Dithiocarbamat-Verbindungen wirkungsvoll gegen Infektionen durch RNA-Viren, die den respiratorischen Trakt befallen und dort eine Erkrankung auslösen - im Rahmen der vorliegenden Anmeldung "respiratorische RNA-Viren" genannt -, eingesetzt werden können. Entgegen den Beobachtungen von Knobil et al. (Am. J. Physiol. (1998), Seiten 134-142) konnte im Rahmen der vorliegenden Erfindung eindeutig eine antivirale Wirkung von den erfindungsgemäßen Dithiocarbamat-Verbindungen bezüglich Infektionen mit respiratorischen RNA-Viren, beispielsweise HRV und Influenza-Infektionen, gezeigt werden. Dies ist umso überraschender, als andere Antioxidantien diese antivirale Wirkung gegenüber respiratorische RNA-Viren nicht aufweisen und die Änderung des Redox-Potentials nicht ausschließlich verantwortlich ist für die antivirale Wirkung von den erfindungsgemäßen Dithiocarbamat-Verbindungen. Beispielsweise kann erfindungsgemäß klar gezeigt werden, dass die Antioxidantien Vitamin C, Vitamin E, 2-Mercaptoethanol und N-Acetyl-L-Cystein keinerlei Wirkung gegen respiratorische RNA-Viren haben. Weiters ist die Effizienz von den erfindungsgemäßen Dithiocarbamat-Verbindungen gegen Infektionen mit respiratorischen RNA-Viren und Vermehrung dieser Viren nicht alleine auf die Inhibition der NFκB-Aktivierung zurückzuführen, sondern es zeigte sich, dass mit den erfindungsgemäßen Dithiocarbamat-Verbindungen, wobei im Rahmen der vorliegenden Anmeldung auch die oxidierten Formen, insbesondere die Dimere, darunter zu verstehen sind, gezielt die Vermehrung von respiratorischen RNA-Viren unterbunden wird.

Die DE 1963223 A betrifft ein Mittel zur Behandlung von Virusinfektionen im Gehirn, wobei das Mittel einen Hemmstoff für die Biosynthese der Monoamine Noradrenalin, Dopamin und 5-Hydroxytryptamin umfassen soll. Das in dieser Schrift angegebene Beispiel zeigt die Wirkung von α-Methyltyrosinmethylester auf Herpes simplex-infizierte Mäuse. Der in dieser Schrift beschriebene Mechanismus der Hemmung der Biosynthese von speziellen Monoaminen ist somit lediglich für die Behandlung von DNA-Virusinfektionen im Gehirn anwendbar. Die erfinderische Behandlung von respiratorischen RNA-Viren funktioniert nach einem anderen Prinzip und ist nicht auf Virusinfektionen im Gehirn anwendbar, wie die Negativbeispiele zu FSME (Beispiel 13) und EMC (Beispiel 14) zeigen. Damit betrifft die DE 1963223 A ein anderes Anwendungsgebiet und ist mit der erfinderischen Anwendung nicht zu vergleichen und legt diese somit auch nicht nahe.

In Calvert J.G., Interferon Research 1990 (10), Seiten 13-23 wird die Wirkung von Diethyldithiocarbamat auf Mengoviren beschrieben, wobei festgestellt wird, dass DDTC Mengovirus-Virionen inaktiviert. Mengoviren sind jedoch Erreger einer schweren Enzephalomyocarditis und befallen nicht den respiratorischen Trakt oder lösen dort eine Erkrankung aus.

Die WO 95/03792 A1 betrifft die Verwendung von Thiolverbindungen für die Herstellung einer pharmazeutischen Zusammensetzung zur Behandlung virus-induzierter Erkrankungen, wobei in Virusproteinen vorhandene Disulfidbrücken durch die Thiolverbindung zerstört werden. In diesem Dokumente werden unter zahlreichen Viren auch RNA-Viren genannt, u.a. Picornaviridae. Weiters werden auch zahlreiche Beispiele von Thiolverbindungen angegeben, u.a. ganz allgemein Dithiocarbamat. Aus diesen vielen verschiedenen Kombinationsmöglichkeiten werden lediglich folgende Beispiele gezeigt: als Thiolverbindungen N-Acetylcystein (NAC), Cystein, Cysteinhydrochlorid und N,S-Diacetylcystein-ethylester (DACEE), als Viruserkrankung sind lediglich Hepatitis B und Vaccinia-Virus gezeigt.

Somit wurden nicht nur für den Großteil der Verbindungen und viruserkrankungen keine Beispiele gezeigt, aber es hat sich auch herausgestellt, dass die in dieser Schrift offenbarte Behandlung nicht in dem Umfang funktioniert, wie sie beschrieben ist: Manche Picornaviren (solche die nicht den respiratorischen Trakt sondern Nervenzellen befallen) werden von PDTC nicht inhibiert oder in ihrer Vermehrung reduziert. Somit sind nicht alle Kombinationen von Virus-induzierten Erkrankungen und Thiolverbindungen zielführend, und die Verwendung der erfindungsgemäßen ausgewählten Dithiocarbamatverbindungen - die in der WO 95/03792 A1 nicht offenbart sind - zur Behandlung oder Verhinderung einer Infektion durch respiratorische RNA-Viren ist nicht nahegelegt.

Die GB 861 043 A betrifft Zusammensetzungen, die u.a. als Schutz gegen Viren eingesetzt werden. Diese Zusammensetzungen umfassen beispielsweise Dithiocarbamate, spezifische Viren werden hier jedoch nicht offenbart.

Knobil et al., Am. J. Physiol. (1998), S. 134-142 betrifft eine Studie zu Oxidantien und deren Einfluss auf Virus-induzierte Genexpression. Hier wird jedoch explizit festgestellt, dass weder NAC noch PDTC eine Influenzavirus-Infektion oder Replikation inhibiert.

In der DE 2555730 A wird ein antimikrobielles Mittel umfassend eine Dimethyldithiocarbamatverbindung beschrieben, wobei diese Verbindung ein 8-Oxychinolinat-Metall-N.N-dimethyldithiocarbamat-Komplex ist. Jedoch wird lediglich die fungizide und antibakterielle Wirkung in dieser Schrift offenbart.

Die WO 99/66918 A1 betrifft die Verwendung von Disulfidderivaten von Dithiocarbamaten zur Reduktion von Stickstoffoxiden in einem Patienten bzw. zur Inhibierung von NFκB. Es wird jedoch in dieser Schrift eine ausgesprochen große Anzahl von Erkrankungen angegeben, wobei die viralen Erkrankungen nicht näher beschrieben werden.

Flory E. et al., J. Biol. Chem., 24.03.2000, 275(12), S. 8307-8314 betrifft eine Studie des Einflusses verschiedener Influenza A-Virusproteine auf die Aktivierung der NFκB abhängigen Expression.

Tai D.I. et al., Hepatology, März 2000, 31(3), S. 785-787 betrifft eine Studie über die Inhibierung der NFxB-Aktivierung durch PDTC, wobei angenommen wird, dass eine HCV-Infektion eine Anti-Apoptose durch Aktivierung von NFκB hervorrufen könnte.

Schwarz et al., 1998 (J. Virol; Vol. 72(7): S. 5654-5660) haben den Einfluss von NFKB auf die Virusvermehrung des dem humanen Rhinovirus verwandten Enzephalomyocarditis-Virus (EMCV) untersucht. In Zellen ohne NFκB (knockout p50-/- oder p65-/-), die mit EMCV infiziert wurden, ist die Virusvermehrung zwar reduziert, jedoch wird verstärkt apoptotischer Zelltod ausgelöst. Dies ist im starken Gegensatz zu den hier dargelegten Daten: Diese Beispiele können klar zeigen, dass die erfindungsgemäßen Dithiocarbamat-Verbindungen nicht nur die Replikation des verwandten Rhinovirus unterbinden, sondern auch den virusinduzierten Zelltod verhindern. Daher ist die Inhibition von NFκB nicht der entscheidende Faktor der Wirksamkeit der erfindungsgemäßen Dithiocarbamat-Verbindungen.

Auch ist die erfindungsgemäße antivirale Wirkung der erfindungsgemäßen Dithiocarbamat-Verbindungen nicht abhängig von einer Kombination von bestimmten Stoffen. Die erfindungsgemäßen Dithiocarbamat-Verbindungen können völlig alleine, unabhängig von den weiteren Zusatzstoffen, insbesondere Antioxidantien, wie dies gemäß der US 5,686,436 für eine anti-Retrovirus Wirkung von NFκB-Aktivierungs-Inhibitoren absolut unerlässlich ist, angewendet werden, weil überraschenderweise die antivirale Wirkung sich nicht nur auf die Bekämpfung des oxidativen Stresses bezieht, sondern die Infektion/Replikation durch die erfindungsgemäßen Dithiocarbamat-Verbindungen inhibiert werden kann.

Es konnte gezeigt werden, dass die erfindungsgemäßen Dithiocarbamat-Verbindungen Gene induzieren, die als Antioxidans induzierte Transkriptionsfaktoren wirken (Meyer et al., EMBO J. 12, 2005-2015, 1993). Der heterodimere Transkriptionsfaktor AP1 kann durch NAC und die erfindungsgemäßen Dithiocarbamat-Verbindungen induziert werden, was zu DNA-Bindung und Transaktivierung führt. Die Aktivierung von AP1 durch die erfindungsgemäßen Dithiocarbamat-Verbindungen ist von der Proteinsynthese abhängig und beinhaltet die Transkription von c-jun und c-fos-Genen. Die Aktivierung von AP1 alleine ist jedoch nicht für die starke erfindungsgemäße Inhibierung der Viren verantwortlich.

Pyrrolidin-Dithiocarbamat (PDTC) ist bereits bekannt als Pro- und Antioxidans, Inhibitor der Aktivierung des Transkriptionsfaktors NFκB, Zink-Ionophor und Metall-Chelatierungs-Agens. In Sherman et al., Biochem. Biophys. Res. Comm., 191 (3):1301-1308, 1993, ist PDTC als Inhibitor der NFKB-Aktivierung sowie der NO-Synthase beschrieben. Die WO 01/00193 A2 betrifft Zusammensetzungen umfassend Diethyldithiocarbamat im picomolaren und nanomolaren Bereich, welche eine starke Wirkung gegen Apoptose aufweisen.

Es konnte nun erfindungsgemäß gezeigt werden, dass die erfindungsgemäßen Dithiocarbamat-Verbindungen eine starke Wirkung gegen RNA-Viren, die den respiratorischen Trakt befallen und dort eine Erkrankung auslösen - "respiratorische RNA-Viren" - sowohl in vitro als auch in vivo aufweisen.

Erfindungsgemäß wird so der Infektion bereits in einer sehr frühen Phase entgegengetreten, noch bevor es zu weitgehenden Zellschäden oder gar Zelltod kommt.

Im Rahmen der vorliegenden Erfindung werden unter respiratorischen RNA-Viren jegliche Viren von Menschen und Säugetieren verstanden, die über den respiratorischen Trakt, also Atemwege und Lunge, den Körper befallen und in den Körper eindringen, wobei sie im respiratorischen Trakt eine Erkrankung auslösen. Offensichtlich sind sich die biologischen Prozesse, die sich bei dieser Infektion ereignen, so ähnlich, dass die Wirkung der erfindungsgemäßen Dithiocarbamat-Verbindungen in analoger Weise effizient wirkt - trotz der biologischen Heterogenität dieser Gruppe von Viren. Umgekehrt hat sich aber auch gezeigt, dass bei anderen Viren, die über andere Infektionswege in den Körper gelangen und andere biologische Zyklen durchlaufen (z.B. als latentes Virus in das Wirtsgenom integriert werden können) bzw. die die Erkrankung in anderen Organen, z.B. im Gehirn, auslösen, die Wirkung der erfindungsgemäßen Dithiocarbamat-Verbindungen alleine zur erfolgreichen Bekämpfung einer Virusinfektion nicht ausreicht.

In diesem Zusammenhang konnte mittlerweile gezeigt werden, dass bei der Behandlung von AIDS-Patienten mit Dithiocarbamaten keine Verbesserung oder Heilung für die Patienten erzielt wird (Multicenter, randomized, placebo-controlled study of dithiocarb (Imuthiol) in human immunodeficiency virus-infected asymptomatic and minimally symptomatic patients. The HIV87 Study Group. AIDS Res. Hum. Retroviruses 1993 Jan; 9(1): 83-9). Aufgrund dieser Ergebnisse wurden keine weiteren klinischen Studien bei latenten Viren durchgeführt (vgl. US 5 686 436).

Ihre besondere Wirksamkeit entfalten die erfindungsgemäßen Dithiocarbamat-Verbindungen gemäß der vorliegenden Erfindung vor allem in einer frühen Phase der viralen Infektion bzw. wenn es vor der Infektion eingenommen wird. So können die erfindungsgemäßen Dithiocarbamat-Verbindungen den Ausbruch einer Virus-Infektion verhindern, wenn es prophylaktisch eingenommen wird, z.B. in Gegenden zu Zeitpunkten, bei welchen ein Risiko oder gar ein erhöhtes Risiko für respiratorische Virus-Infektionen besteht, etwa in Regionen mit Epidemien oder von Kältewellen. Bevorzugterweise werden dabei die erfindungsgemäßen Dithiocarbamat-Verbindungen zur Verhinderung der Virus-Infektion verwendet.

Ganz besonders bevorzugt ist die erfindungsgemäße Virusinhibierung aber vor allem in der Frühphase einer bereits erfolgten respiratorischen RNA-Virus-Infektion. Dabei werden die erfindungsgemäßen Dithiocarbamat-Verbindungen gezielt zur Inhibierung der Replikation der Viren eingesetzt, also zu einem Zeitpunkt bevor noch nachhaltige Schäden im infizierten Individuum aufgetreten sind. Damit können nicht nur die Folgen einer fortschreitenden Virus-Infektion im betroffenen Individuum selbst verhindert werden, sondern auch die Verbreitung weiterer infektiöser Viren an andere Individuen; die weitere Ansteckungsgefahr wird minimiert, was besonders bei menschlichem Influenza-Virus eine große allgemein gesundheitspolitische Wirkung und Bedeutung hat.

Insbesondere gelten als respiratorische RNA-Viren im Rahmen der vorliegenden Erfindung: Rhinoviren, Coxsackieviren, Echoviren, Coronaviren, Enteroviren, humane Orthomyxoviren (z.B. Influenza Virus (A, B und C)), Paramyxoviren (z.B. Parainfluenzavirus und Pneumovirus), respiratorischer Syncytialvirus (RSV) und andere RNA-Viren, solange sie eine Erkrankung (zumindest) im respiratorischen Trakt auslösen. Viren oder Virusstämme, die keine Erkrankung im respiratorischen Trakt sondern in einem anderen Organ, etwa im Gehirn, auslösen, z.B. Meningitis-Virus, Enzephalomyocarditis-Virus, Poliovirus, Cardiovirus, etc., sind von der vorliegenden Anmeldung nicht umfasst. Den erfindungsgemäßen respiratorischen RNA-Viren ist die Infektion von epithelialen zellen des oberen oder unteren respiratorischen Trakts gemeinsam. Weiters können auch zusätzlich andere Organe befallen werden. Die dadurch verursachte lokale Inflammation im respiratorischen Trakt gilt als die Hauptursache für die Entstehung der typischen Symptome eines grippalen Infekts, wie Schnupfen, Halsweh, Heiserkeit, Husten, Bläschen bzw. häufig Fieber. Auch das häufige Auftreten von Sekundärinfektionen im immungeschwächten Individuum wird durch die Infektion mit diesen respiratorischen Viren begünstigt.

Im Rahmen der vorliegenden Erfindung werden unter Picornaviren sämtliche "echte" Picornaviren verstanden, gemäß der derzeit gültigen Klassifikation der Picornaviridae auf Basis von King et al. ("Picornaviridae" in "Virus Taxonomy, Seventh Report of the International Committee for the Taxonomy of Viruses" (2000), Eds. Van Regenmortel et al., Academic Press 657-673), soweit sie den respiratorischen Trakt befallen und dort eine Erkrankung auslösen, d.h. die Genera Enterovirus, Rhinovirus, Aphthovirus, Parechovirus, Erbovirus, Kobuvirus und Teschovirus. Diese Viren der Familie Picornaviridae zeichnet eine ähnliche genetische Struktur, Proteinzusammensetzung, Kultivierungseigenschaften oder Resistenzvermögen gegen Temperatur oder viruziden Mitteln aus.

Es zeigte sich, dass sich die vorliegende Erfindung besonders gut zur Bekämpfung der human- und tierpathogenen Vertreter der echten respiratorischen Picornaviridae, insbesondere aus den Genera Enterovirus (Enterovirus 70, 71, Coxsackievirus) und Rhinovirus (z.B. humanes Rhinovirus) und Aphthovirus (z.B. Maul- und Klauenseuchevirus) eignet, wohingegen für andere Viren, inklusive der Picornaviren, welche latente Infektionen verursachen wie z.B. HAV, die erfindungsgemäßen Vorteile nicht belegt werden konnten. Dies ist wahrscheinlich auch darauf zurückzuführen, dass die Gruppe der "echten" respiratorischen Picornaviren in sich selbst so homogen ist und dass die patho-physiologischen Prozesse, die im Rahmen der Infektionen auftreten, so ähnlich sind, dass die erfindungsgemäßen Dithiocarbamat-Verbindungen in analoger Weise zur Wirkung kommen.

Unter "Virus-Infektion" wird im Rahmen der vorliegenden Anmeldung jeglicher Angriff eines respiratorischen Virus auf Zellen verstanden, der beispielsweise einen der folgenden Schritte umfasst: Beginnend mit dem Andocken des Viruspartikels an eine Zelle, und in einem späteren Stadium das Einführen der genetischen Information des Virus in die Zelle sowie die Produktion von neuen Viruspartikelteilen und die Expression von infektiösen Viruspartikeln.

Es ist weiters auch möglich, oxidierte Formen dieser Verbindungen, insbesondere Dimere hievon, zu verwenden, da diese wie an sich bekannt im Organismus rasch in die reduzierte Form metabolisiert werden. Im Rahmen der vorliegenden Anmeldung sind solche Verbindungen unter "oxidierte Formen" zu verstehen, deren S-Rest oxidiert ist. Ein bevorzugtes Beispiel einer solchen oxidierten dimeren Form stellt das Disulfiram dar, das auch unter dem Namen "Antabus" oder "Abstinyl" bekannt ist und ein Tetraethylthiuramdisulfid (C₁₀H₂₀N₂S₄) ist. Das Disulfiram an sich ist bereits bekannt, wobei es insbesondere zur Behandlung von Alkoholikern verwendet wird: Disulfiram wirkt als Mediator bei Oxidoreduktionen und inaktiviert die Aldehyd-Dehydrogenase. Durch die Einnahme von Ethanol kommt es zur Anreicherung von Acetaldehyd im Körper, was eine ausgesprochen negative Wirkung auf das allgemeine Wohlbefinden ausübt: Bei gleichzeitiger Einnahme von Disulfiram und Alkohol kommt es zu Angstzuständen, Übelkeit, Sehverlusten, Brustschmerzen, Kopfschmerzen etc., wobei diese Symptome 3-4 Tage bzw. auch eine Woche lang anhalten. Aus diesem Grund wird Disulfiram Alkoholikern als Therapie verabreicht, da jeglicher nachfolgende Alkoholkonsum auf Grund dieser starken negativen Effekte vermieden werden sollte.

Andere bekannte Wirkungen von Disulfiram sind die Inhibierung von Enzymen wie beispielsweise Fructose, 1,6-Diphosphat-Dehydrogenase, Xanthin-Oxidase, Hexokinase, Aldehyd-Oxidase.und Dopamin-β-Hydroxylase.

Weiters wird Disulfiram zur Behandlung von Pediculosis und Scabies sowie auch zur Behandlung von Nickel-Dermatitis eingesetzt.

Der Metabolismus von Disulfiram wurde bereits eingehend analysiert, s. beispielsweise Dollery, C. 1999, Therapeutic Drugs, Second Edition, Vol.1, Churchill Livingstone, Edinburgh. Es ist beschrieben, dass Diethyldithiocarbamat den Hauptmetabolit von Disulfiram im Körper darstellt, wobei diese Umwandlung sehr rasch vor sich geht.

Die oxidierte Form der erfindungsgemäßen Dithiocarbamat-Verbindungen ist gut fettlöslich und kann beispielsweise als oral zu verabreichendes Mittel vorgesehen werden, wobei die Verbindungen im Magen resorbiert werden. Die Verbindungen könnten dabei insbesondere auch als Pulverspray vorgesehen werden. Weiters können die oxidierten Form der erfindungsgemäßen Verbindungen mit Hydroxylgruppen versehen werden, um die Wasserlöslichkeit zu erhöhen, um es in Form eines Aerosols zur Verfügung zu stellen. Werden die erfindungsgemäße Dithiocarbamat-Verbindungen in Form des oxidierten, insbesondere dimeren Produkts vorgesehen, wird eine Depotwirkung dieses Mittels erzielt, d.h. die erfindungsgemäße Dithiocarbamat-Verbindungen werden über eine bestimmte Zeit hinweg allmählich an den Körper abgegeben und von diesem resorbiert. Für diese Depotwirkung können die oxidierten Dithiocarbamat-Verbindungen auch in den zu behandelnden Körper wie an sich bekannt implantiert werden.

Eine bevorzugte erfindungsgemäße Verbindung ist dadurch gekennzeichnet, dass R₁ und R₂ unabhängig voneinander ein C₁-C₃-Alkyl darstellen oder mit dem Stickstoffatom einen aliphatischen Ring mit 4 bis 6 C-Atomen bilden. Diese haben sich als besonders vorteilhaft für die Behandlung oder Verhinderung einer respiratorischen RNA-Virusinfektion herausgestellt.

Vorzugsweise ist die Dithiocarbamat-Verbindung ausgewählt aus Pyrrolidin-Dithiocarbamat (PDTC) und N,N-Diethyl-Dithiocarbamat (DDTC). Diese Verbindungen weisen eine ausgesprochen starke Aktivität gegen respiratorische RNA-Virusinfektionen auf.

Besonders bevorzugt ist die Virus-Infektion eine Infektion mit Picornavirus, Orthomyxo- oder Paramyxovirus. Die erfindungsgemäßen Dithiocarbamat-Verbindungen sind besonders aktiv gegen diese Virus-Infektionen.

Von Vorteil ist dabei, wenn das Orthomyxovirus ein humanes Influenzavirus ist, insbesondere ausgewählt aus der Gruppe bestehend aus Influenza A, Influenza B, Influenza C bzw. das Paramyxovirus ein Parainfluenzavirus oder Pneumovirus ist. Vorzugsweise ist das Orthomyxovirus ein Säugetier-Influenza A-Virus und das Picornavirus ein Rhinovirus, insbesondere humanes oder Pferde-Rhinovirus, ein Enterovirus, insbesondere Enterovirus 70, 71 oder Coxsackievirus, bzw. ein Aphthovirus, insbesondere das Maul- und Klauenseuche-Virus oder equines Rhinitisvirus A, ist. Dabei sind, wie oben bereits erläutert, solche Viren oder Virusstämme ausgenommen, die keine Erkrankung im respiratorischen Trakt, sondern etwa im Gehirn oder in Nervenzellen auslösen, z.B. Cardioviren oder Polioviren, die an sich zu den Picornaviren zählen. Diese Virus-infektionen werden besonders effizient mit der erfindungsgemäßen Verbindung behandelt bzw. verhindert.

Die Familie der Picornaviridae umfasst eine Reihe von kleinen RNA-Viren unter anderem Rhinoviren (z.B. das humane Rhinovirus), Enteroviren (z.B. Enterovirus 70, 71, Coxsackievirus, das beispielsweise ein Erreger der Hand-, Fuß- und Mundkrankheit ist), Aphthoviren (z.B. das Maul-und Klauenseuche-Virus, wobei hier eine Erkrankung im Mund hervorgerufen wird, sowie equines Rhinitisvirus A). Betreffend z.B. Maul- und Klauenseuche ist besonders interessant, mit Hilfe der erfindungsgemäßen Verbindung den Zeitpunkt zwischen der Impfung und dem Wirksamwerden des Impfstoffes zu überbrücken (ca. 10-12 Tage), da während dieser Zeit die geimpften Tiere nicht geschützt sind und unter Umständen nach einer Infektion große Mengen von Virus ausscheiden, ohne selbst zu erkranken. Somit könnte die erfindungsgemäße Verbindung den Tieren gleichzeitig mit der Impfung und eventuell danach ca. 2 Wochen lang verabreicht werden.

Orthomyxo- und Paramyxoviren sind eine Unterteilung der früheren Sammelbezeichnung für Influenzaviren und anderen ähnlichen Viren. Beim Menschen rufen Paramyxoviren Masern, Mumps, respiratorische und neurologische Erkrankungen hervor. Zu den Paramyxoviren gehören unter anderem das Parainfluenzavirus, Mumpsvirus, Newcastle disease-virus, das respiratorische Syncytialvirus (RSV), das Masernvirus und das Rinderpestvirus. Zu den Orthomyxoviren gehören unter anderem das Influenzavirus A, B und C, die die Erreger der Grippe beim Menschen sind.

Die Influenzaviren vom Typ A sind verantwortlich für die Mehrzahl der Grippeepidemien sowie für alle Pandemien. Influenza A-Viren kommen zwar auch bei Pferden und Schweinen vor, des weiteren auch bei Vögeln, beispielsweise als Erreger der klassischen Geflügelpest, jedoch können nur die menschlichen Influenza-Viren und die Influenza-Viren von Säugetieren, z.B. Pferd, als respiratorische Viren im eigentlichen Sinn gelten, da sich die Biologie vom Vögel-Influenza-Virus (Avian Influenza Virus (AI)) völlig vom menschlichen Influenza-Virus unterscheidet. Daher kann gerade das AI-Virus nicht als respiratorisches Virus angesehen werden. In Enten repliziert das AI-Virus primär im Intestinaltrakt, was beim Menschen nicht der Fall ist. Als Konsequenz können AI-Viren auch aus faeces von Vögeln isoliert werden. (Hinshaw et al., 1980, Canad. J. Microbiol. 26, 622-9). Weiters ist die Nukleotid-Variationsrate von AI-Viren niedriger als jene bei Viren, die von Säugetieren isoliert werden können. Die Evolution der viralen Proteine in anderen Organismen als Vögeln zeigt typischerweise eine rapide Akkumulation von Mutationen, die bei AI-Viren nicht vorkommen. (Gorman et al., 1991, J. Virol. 65: 3704-14; Ludwig et al., 1995, Virology 212: 555-61). Die Rezeptorspezifität variiert zwischen verschiedenen Influenza-Viren. Die meisten AI-Viren binden vorzugsweise an den alpha2-3-Galactose-Sialinsäurerezeptor. Im Gegensatz dazu binden humane Influenza-Viren primär an den alpha2-6-Galactose-Sialinsäurerezeptor (Rogers + Paulson, 1983, Virology 127, 361-73; Baum + Paulson, 1990, Acta Histochem. Suppl. 40: 35-8).

Da die erfindungsgemäßen Viren bei Mensch und Säugetieren zu einer Anzahl von weit verbreiteten Krankheiten führen, ist die antivirale Wirkung der erfindungsgemäßen Dithiocarbamat-Verbindungen im Hinblick auf diese Viren besonders wichtig. Da die erfindungsgemäßen Dithiocarbamat-Verbindungen ausgesprochen wirksam gegen diese Viren sind, eignen sich die erfindungsgemäßen Dithiocarbamat-Verbindungen insbesondere zur Herstellung von einer Reihe von Mitteln zur Behandlung bzw. Prävention dieser Virus-Infektionen. Die erfindungsgemäßen Dithiocarbamat-Verbindungen sind leicht und kostengünstig in großen Mengen herstellbar und wirken auch in höheren Konzentrationen kaum toxisch auf die zu behandelnden Zellen.

Gemäß einer vorteilhaften Ausführungsform sind die erfindungsgemäßen Dithiocarbamat-Verbindungen in dem Mittel in einer Konzentration von 0,01 bis 5000 mM, vorzugsweise 1 bis 300 mM, besonders bevorzugt 10 bis 100 mM, vorgesehen. In diesen Konzentrationen sind die erfindungsgemäßen Dithiocarbamat-Verbindungen besonders wirksam gegen respiratorische RNA-Virusinfektionen und weisen kaum bzw. keine Nebeneffekte auf. Die einzusetzende Konzentration wird je nach der zu behandelnden Virus-Infektion, je nach Stärke der Virus-Infektion bzw. je nach dem zu behandelnden Organismus, etwa ob Tier oder Mensch bzw. in Abhängigkeit des Alters, ausgewählt.

Besonders günstig ist es, wenn die erfindungsgemäßen Dithiocarbamat-Verbindungen in dem Mittel in einer Konzentration von 10 mM bis 1 M vorgesehen sind. In diesem Fall liegen die erfindungsgemäßen Dithiocarbamat-Verbindungen in hoch konzentrierter Form vor und das Mittel kann vor der Behandlung je nach gewünschter Konzentration verdünnt werden.

Vorzugsweise umfasst das Mittel weiters einen pharmazeutisch akzeptablen Träger. Dabei kann jeder dem Fachmann auf dem Gebiet der Pharmazie bekannte pharmazeutisch akzeptable Träger eingesetzt werden, wie zum Beispiel Phosphat-gepufferte Salzlösung (PBS) oder anders gepufferte Kochsalzlösungen bzw. Liposomen-hältige Formulierungen, wobei hier wiederum der Träger je nach Behandlungsart, Virus-Infektion bzw. dem zu behandelnden Organismus ausgewählt wird.

Vorzugsweise ist das Mittel ein oral, intranasal, intravenös, parenteral, rektal bzw. als Augen- oder Ohrentropfen, Gurgellösung oder Aerosol zu verabreichendes Mittel. Dabei hängt die Art der Verabreichung insbesondere von der zu behandelnden Virus-Infektion ab. Beispielsweise wird eine Infektion im respiratorischen Trakt, z.B. durch ein intranasal zu verabreichendes Mittel, z.B. in Form eines Aerosols umfassend die Dithiocarbamat-Verbindungen, behandelt werden, da die Virus-Infektion gleich am Ort des Virusangriffs behandelt wird. Je nach Art der Verabreichung liegen die Dithiocarbamat-Verbindungen in einer bestimmten Konzentration vor bzw. umfasst das Mittel zusätzliche für diese Verabreichungsform günstige Substanzen. Es ist selbstverständlich möglich, das Mittel etwa in getrockneter Form vorzusehen, wobei es vor der Behandlung mit einem geeigneten Lösungsmittel verdünnt wird.

Eine besonders vorteilhafte Verwendung ist gegeben, wenn das Mittel weitere antivirale Substanzen umfasst. Auf diese Weise kann die Virus-Infektion im respiratorischen Trakt von mehreren Seiten her bekämpft werden bzw. es kann gleichzeitig eine ganze Palette von verschiedenen Viren abgeschwächt bzw. vollständig ausgeschaltet werden. Solche weiteren antiviralen Substanzen sind etwa Substanzen, die die Replikation hemmen, immun-stimulierende Substanzen, neutralisierende Antikörper etc., aber auch gegebenenfalls Substanzen, die das Immunsystem generell unterstützen können.

Vorzugsweise umfasst das Mittel eine Kombination von zumindest zwei verschiedenen erfindungsgemäßen Dithiocarbamaten, insbesondere eine Mischung aus PDTC und DDTC. Die erfindungsgemäßen Dithiocarbamat-Verbindungen üben pro- und anti-oxidative Funktionen in Zellen aus. Ihre anti-oxidative Wirkung umfasst die Eliminierung von Wasserstoffperoxid, Entfernen von Superoxid-Radikalen, Peroxynitriten, Hydroxylradikalen und Lipid-Peroxidationsprodukten. Durch diese Eliminationen werden die Dithiocarbamate zu Thiuram-Disulfid oxidiert. Thiuram-Disulfide sind verantwortlich für die pro-oxidativen Wirkungen von Dithiocarbamaten, wobei in manchen Fällen die Bildung von Thiuramen abhängig von der Gegenwart von Metallen ist. Es wurde beschrieben, dass die anti-apoptotischen Aktivitäten von Dithiocarbamaten möglicherweise in Verwendung mit der Inaktivierung von Kaspasen durch Thiol-Oxidation stehen.

Besonders bevorzugt umfasst das erfindungsgemäße Mittel weiters Substanzen, ausgewählt aus Antibiotika, Impfstoffen, Immunsuppressiva, Stabilisatoren, immunstimulierenden Substanzen, Blutprodukten oder Mischungen davon. Werden zusätzlich Antibiotika eingesetzt, können neben den respiratorischen Viren weiters Bakterien-Infektionen bekämpft werden. Umfasst das Mittel zusätzliche Impfstoffe, wobei hierunter sowohl passive als auch aktive Impfstoffe zu verstehen sind, werden gleichzeitig mit der erfindungsgemäßen Behandlung bzw. Prävention der Virus-Infektion auch bestimmte andere weitere Virus-Infektionen verhindert, die den geschwächten Organismus leicht infizieren können. Zusätzlich können zur Erhöhung der Lagerungsstabilität bzw. Lebensdauer weiters Stabilisatoren zugesetzt werden. Blutprodukte sind z.B. Plasma, Blutkörperchen, Gerinnungsfaktoren, etc., je nachdem, welcher Behandlung der Patient unterzogen wird.

Günstigerweise wird das Mittel zur Inhibierung der Virusvermehrung eingesetzt. Auf diese Weise ist gesichert, dass sich eine bereits erfolgte Infektion nicht ausbreitet, sondern im Gegenteil sehr rasch behandelt wird.

Gemäß einem weiteren Aspekt der vorliegenden Erfindung betrifft die Erfindung auch ein Desinfektionsmittel umfassend zumindest eine erfindungsgemäße Dithiocarbamat-Verbindung wie oben beschrieben. Unter "Desinfektionsmittel" wird im Rahmen der vorliegenden Erfindung jegliches Mittel verstanden, das außerhalb eines menschlichen oder tierischen Organismus zur Bekämpfung von Viren eingesetzt wird, etwa auf Oberflächen, in Mitteln, insbesondere Medien, oder für Zellkulturen. Solche Desinfektionsmittel können insbesondere dann eingesetzt werden, wenn die zu behandelnde Substanz empfindlich ist gegenüber anderen aggressiveren antiviralen Stoffen. Beispielsweise eignen sich Desinfektionsmittel umfassend die erfindungsgemäßen Dithiocarbamat-Verbindungen als Zusatz für Medien bzw. zur Behandlung von Zellen oder Zellkulturen, die auf andere, aggressivere Desinfektionsmittel bzw. antivirale Substanzen empfindlich reagieren. Die erfindungsgemäßen Dithiocarbamat-Verbindungen haben sich als besonders wirksam bei der Behandlung bzw. Prävention von respiratorischen RNA-Virus-Infektionen von respiratorischen Zellen und Zellkulturen gezeigt.

Besonders wirksam ist das Desinfektionsmittel, wenn es die erfindungsgemäßen Dithiocarbamat-Verbindungen in einer Konzentration von 10 µM bis 5 M, insbesondere 30 µM bis 1 M, umfasst. Diese Konzentrationen sind einerseits wirksam gegen respiratorische RNA-Virus-Infektionen, andererseits ist ein solches Desinfektionsmittel ausgesprochen schonend, beispielsweise wenn es als antivirale Substanz für Zellkulturen eingesetzt wird. Die Konzentration hängt dabei von der Art bzw. vom fortgeschrittenen Stadium der Virus-Infektion ab bzw. von der zur Behandlung verwendeten Substanz, beispielsweise von der Art und Empfindlichkeit der Zellen. Selbstverständlich ist es auch möglich, das Desinfektionsmittel als Konzentrat vorzusehen, wobei es vor Verwendung mit einem geeigneten Lösungsmittel auf die gewünschte Konzentration der erfindungsgemäßen Dithiocarbamat-Verbindungen gebracht wird.

Vorzugsweise umfasst das Desinfektionsmittel weitere desinfizierende, insbesondere antivirale Stoffe. Diese jedem Fachmann auf dem Gebiet der Mikrobiologie bekannten Stoffe werden insbesondere dann zugesetzt, wenn andere Viren gleichzeitig bekämpft werden sollen, etwa DNA-viren. Selbstverständlich können weiters auch antibakterielle Substanzen, insbesondere Antibiotika, zugesetzt werden.

Ein weiterer Aspekt der vorliegenden Erfindung betrifft ein Verfahren zur Desinfektion von Oberflächen, Medien bzw. Zellkulturen, wobei ein erfindungsgemäßes Desinfektionsmittel wie oben beschrieben auf die Oberfläche bzw. zellkultur aufgebracht bzw. in das Medium eingebracht wird. Zur Desinfektion einer Oberfläche genügt es beispielsweise die Oberfläche mit dem Desinfektionsmittel zu reinigen. Im Fall von Medien und Zellkulturen kann das Desinfektionsmittel über längere Zeit hinweg einwirken, wobei die Konzentration des Desinfektionsmittels wie oben beschrieben je nach Zweck variiert werden kann.

Gemäß einem weiteren Aspekt der vorliegenden Erfindung betrifft die Erfindung die Behandlung bzw. Prävention einer respiratorischen RNA-Virus-Infektion mit den erfindungsgemäßen Dithiocarbamat-Verbindungen. Dabei wird ein Mittel umfassend die erfindungsgemäßen Dithiocarbamat-Verbindungen wie oben beschrieben in einer geeigneten Form und in einer geeigneten Konzentration dem Patienten bzw. dem Tier verabreicht.

Die vorliegende Erfindung wird nun anhand der nachstehenden Beispiele und Figuren näher erläutert, auf die sie jedoch nicht beschränkt ist.
Fig.1 ist eine graphische Darstellung, die die inhibitorische Wirkung von PDTC auf die HRV-Replikation in Zellkulturen zeigt;
Fig.2 zeigt die Inhibition von durch Rhinoviren induzierten cytopathischen Effekte durch PDTC;
Fig.3 zeigt die Erhöhung der Zelllebensfähigkeit durch PDTC und DDTC;
Fig.4 zeigt die Wirkung einer PDTC-Behandlung in Abhängigkeit von der Zeit;
Fig.5 zeigt die Spaltung von eIF4GI;
Fig.6 zeigt eine Western Blot-Analyse zur Bestimmung der Expression von Rhinovirus-Kapsidproteinen;
Fig.7 zeigt den Effekt von anderen Antioxidantien auf die HRV-Infektion;
Fig.8 und 9 zeigen den Effekt von PDTC auf die Influenzavirus-Replikation;
Fig. 10 zeigt die Konzentrationsabhängigkeit der Wirkung von PDTC auf mit Influenza-Viren infizierte Verozellen;
Fig. 11A und 11B zeigen die Wirkung von PDTC auf mit Influenza-virus infizierte Mäuse;
Fig.12 zeigt die Wirksamkeit von PDTC gegen ERAV;
Fig. 13A und 13B zeigen die Wirksamkeit von PDTC gegen MKS; und
Fig. 14 zeigt die fehlende Wirkung von PDTC gegen EMCV.

### Beispiele

### Beispiel 1: Reduktion der Produktion von infektiösen Rhinovirus-Partikeln durch PDTC

Um die Wirksamkeit während Infektionen von HRV zu testen, wurde PDTC nach Infektion von Zellen mit unterschiedlichen HRV-Serotypen zugesetzt.

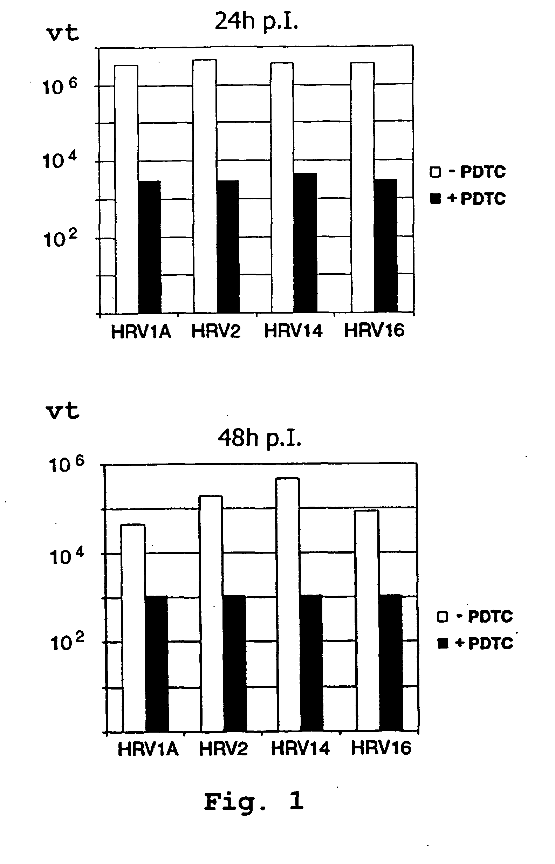

HeLa-Zellen wurden mit HRV-Serotypen 1A, 2, 14 und 16 mit 20 TCID₅₀ (tissue-culture-infectious-dose 50) pro Zelle infiziert. Gleichzeitig wurde PDTC in einer Konzentration von 125 µM dem Medium zugesetzt. Überschüssiges Virus wurde 4h nach Infektion entfernt, während PDTC erneut dem frischen Medium zugegeben wurde. 24 Stunden (Fig.1, oben) und 48 Stunden (Fig.1, unten) nach der Infektion (p.I.) wurden die Überstände gesammelt und die Menge der Virusnachkommen durch TCID₅₀-Tests bestimmt. Die Behandlung von Zellen mit PDTC verringert den Virustiter (vt) um 10³ nach 24 h (Fig. 1, oben). Die Überstände von PDTC behandelten Zellen, die 48 Stunden nach der Infektion gesammelt wurden, zeigen ebenfalls eine signifikante Reduktion des Virustiters (Fig.1, unten). Diese Tests zeigen, dass PDTC stark antiviral gegen verschiedene HRV-Serotypen wirkt.

### Beispiel 2: PDTC inhibiert HRV-induzierte cytopathische Effekte und erhöht die Lebensfähigkeit von infizierten Zellen

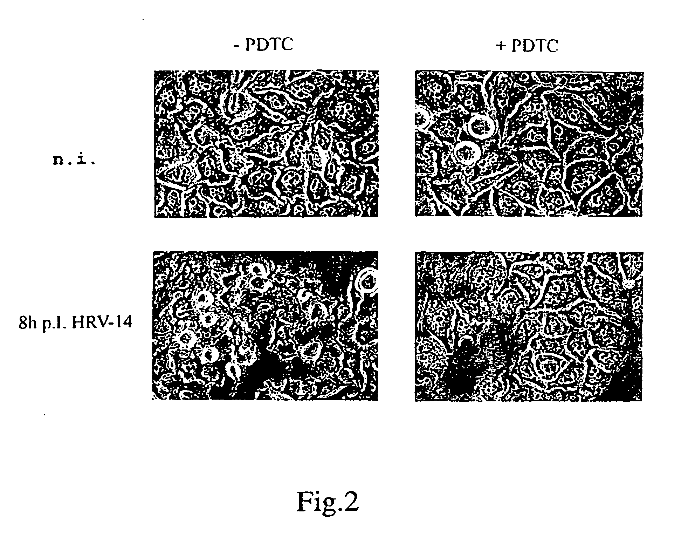

In den späten Stadien einer rhinoviralen Infektion treten morphologische Änderungen der Zellen auf, bekannt als "cytopathische Effekte" (CPE). Diese durch HRV induzierten CPE sind charakterisiert als Zellrundung, Schrumpfen, Deformation des Kerns und Chromatin-Kondensation. Bei einer Infektion von HeLa-Zellen mit HRV2, HRV14, HRV1A und HRV16 mit 100 TCID₅₀ pro Zelle ist ein eindeutiger cytopathischer Effekt nach 8 h p.I. sichtbar. Der Zusatz von PDTC während der Infektion inhibiert das Auftreten dieser cytopathischen Effekte. In Fig.2 ist gezeigt, dass die Morphologie der Zellen 8 h nach der Infektion in Gegenwart von 125 µM PDTC (s. rechte untere Figur) nicht zu unterscheiden ist von der Morphologie von nicht-infizierten (n.i.) Zellen (s. die zwei oberen Figuren).

Die Behandlung mit PDTC führt somit zu einer Bekämpfung der Virus-Infektion in einem sehr frühen Stadium, so dass weitergehende Zellschäden verhindert werden.

### Beispiel 3: Erhöhung der Lebensfähigkeit von infizierten Zellen durch PDTC und DDTC

Ein Zellen-Proliferations-Assay wurde durchgeführt, um den Effekt von PDTC oder DDTC auf die Lebensfähigkeit der Zellen während einer viralen Infektion zu testen.

Dazu wurde ein Zell-Titer 96® Aqᵤₑₒᵤₛ Non-radioactive Cell Proliferation Assay (Promega; Madison, Wisconsin, U.S.A) gemäß der Anleitung des Herstellers durchgeführt. Einen Tag vor der Infektion wurden Zellen in 96 Well-Platten gesetzt. Diese Zellen wurden mit verschiedenen HRV-Serotypen mit 20 TCID₅₀/Zelle infiziert. Die Lebensfähigkeit der Zellen wurde durch Zusetzen einer Tetrazoliumsubstanz, 2 h Inkubation bei 37°C und Messen der Absorption bei 492nm bestimmt .

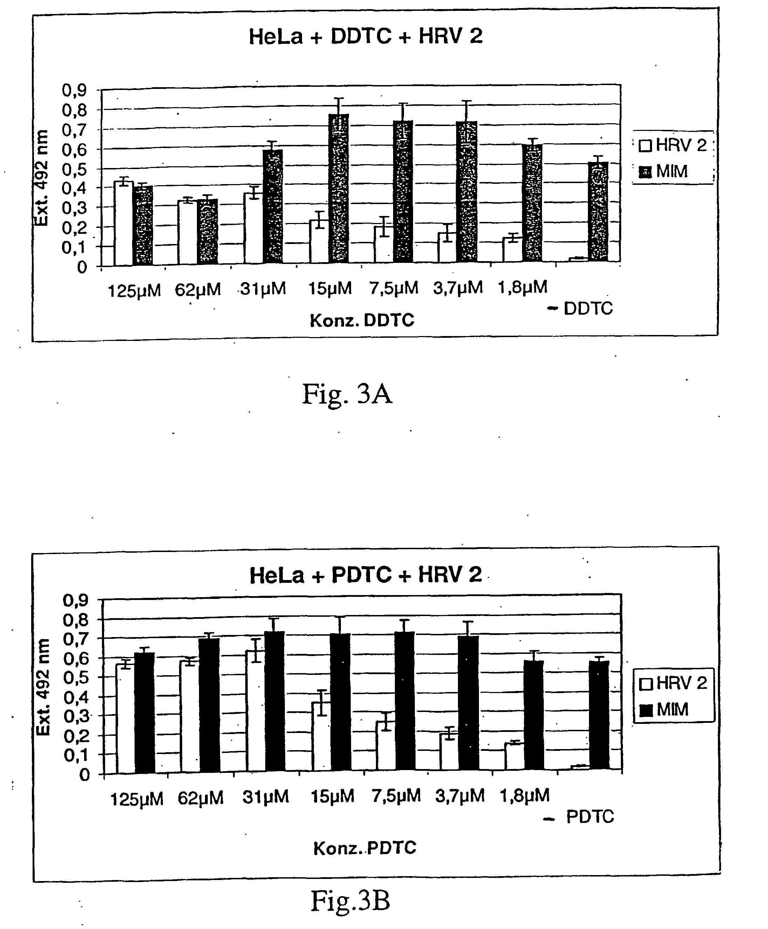

24 h nach der Infektion mit HRV2 zeigten HeLa-Zellen im Vergleich zu nicht-infizierten HeLa-Zellen (mock infection medium = MIM) einen vollständigen Verlust ihrer metabolischen Aktivität. In Fig.3 (oben: DDTC; unten PDTC) ist ersichtlich, dass die Lebensfähigkeit der Zellen selbst bei Zusatz von geringen Konzentrationen von PDTC oder DDTC signifikant erhöht ist. PDTC oder DDTC alleine hat nur geringe Auswirkungen auf nicht infizierte Zellen. Die Konzentrationen, die zur Inhibition der viralen Infektionen eingesetzt werden, zeigten jedoch keine Toxizität ("-" bedeutet ohne).

### Beispiel 4: Wirksamkeit von PDTC in Abhängigkeit von der Zeit

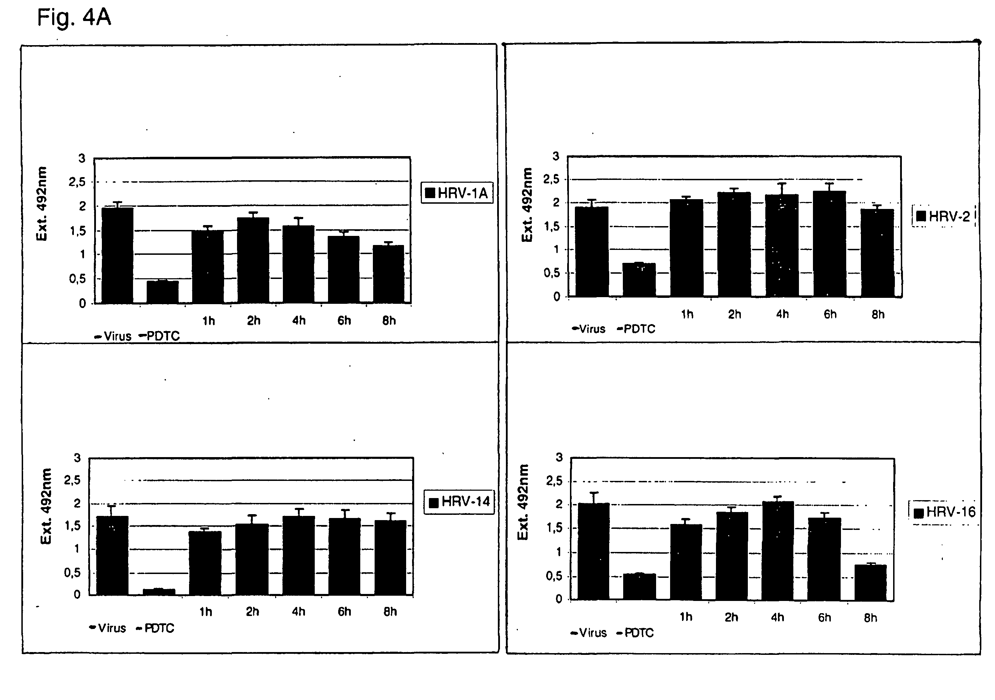

Um zu testen, in welchem Stadium des viralen Lebenszyklus PDTC eingreift, wurde PDTC zu verschiedenen Zeitpunkten nach Virus-Infektion mit 20 TCID₅₀ pro Zelle zugesetzt und es wurden Proliferation-Assays durchgeführt. Es konnte gezeigt werden, dass der Zusatz von PDTC (125 µM) innerhalb der ersten sechs Stunden nach Infektion ("-" bedeutet ohne) den besten Schutz vor Virus-induziertem Verlust der Proliferation bietet (Fig. 4A). Auch dieser Effekt ist nicht spezifisch für einen einzigen Serotyp, da HRV-Serotypen 1A, 2, 14 und 16 verwendet wurden. Erst die PDTC-Behandlung (125 µM) von Zellen zu einem Zeitpunkt später als 8 Stunden nach Infektion verringerte die Schutzwirkung.

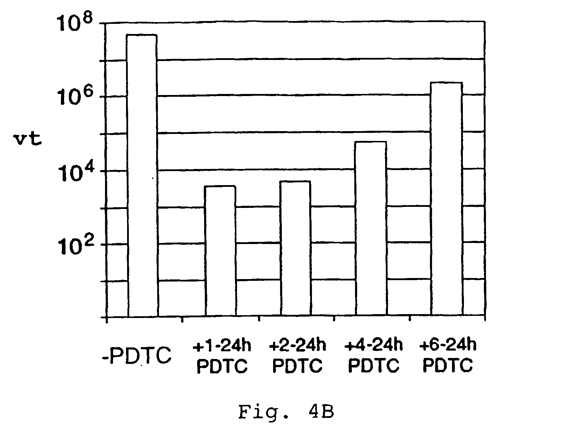

Ähnliche Ergebnisse wurden erhalten, wenn die viralen Titer (vt) in den überständen von infizierten und PDTC-behandelten Zellen bestimmt wurden (s. Fig.4B). Der Zusatz von PDTC bis zu vier Stunden nach Infektion reduzierte die Titer von produziertem HRV2 um 10³. Selbst wenn die PDTC-Behandlung erst 6 Stunden nach Infektion begonnen wurde, wurden die produzierten viralen Titer signifikant verringert. Diese Ergebnisse zeigen, dass der Zusatz von PDTC auch dann einen antiviralen Effekt hat, wenn dies zu späteren Stadien während der Infektion geschieht, wobei sowohl die Lebensfähigkeit der Zellen erhöht wird, als auch die Menge an infektiösen Viren dramatisch verringert wird.

Damit wurde gezeigt, dass die antivirale Wirkung nicht nur in den frühen Stadien des viralen Lebenszyklus auftritt, beispielsweise während der Rezeptorbindung bzw. dem Eintritt in die Zelle.

### Beispiel 5: Untersuchung des HRV-Infektionsverlaufes in Anwesenheit von PDTC

Um den Verlauf einer rhinoviralen Infektion in Gegenwart und Abwesenheit von PDTC zu überprüfen, wurden verschiedene proteolytische Aktivitäten analysiert. Eine charakteristische proteolytische Aktivität, die im Lauf von Infektionen mit Rhino- und Enteroviren auftritt, ist das enzymatische Spalten der zellulären Translations-Initiationsfaktoren 4GI (eIF4GI) und 4GII mit der viralen 2A-Proteinase, so dass die Wirtszellen-Proteinsynthese beendet wird. Je nach HRV-Serotyp werden eIF4G-Proteine bereits in einem frühen Stadium einer Infektion gespalten. Eine weitere, erst kürzlich beschriebene Spaltungsaktivität während einer rhinoviralen Infektion ist das Spalten des intermediären Filament-Proteins Cytokeratin 8. Auch diese Spaltung ist abhängig von der 2A-Protease, tritt aber zu einem späten Stadium während der Infektion auf. Um den Einfluss einer PDTC-Behandlung auf den Verlauf einer Virus-Infektion zu testen, wurde eine Western-Blot-Analyse durchgeführt, um diese 2A-Protease-Substrate zu analysieren.

Für die Western Blot-Analyse wurde zu den angezeigten Zeitpunkten Medium entfernt. Die Zellen wurden durch Zusatz von 100 µl Protein-Puffer (8% Natrium-Dodecylsulfat, 20% β-Mercaptoethanol, 20% Glycerol, 0,04% Bromphenolblau) lysiert. 20 µl Proteinextrakt wurden pro Spur mittels SDS-PAGE aufgetrennt und auf PVDF-Membrane geblottet. Die Inkubation mit Antikörpern wurde mit 0,1% Tween 20 und 5% Magermilchpulver in TBS durchgeführt. Für die Immunodetektion wurden polyklonale Kaninchen-Antikörper gegen eIF4GI eingesetzt. Als sekundäre Antikörper wurden Anti-Kaninchen-Immunglobuline konjugiert mit alkalischer Phosphatase verwendet. Die Färbung wurde durch die alkalische Phosphatasereaktion durchgeführt, die molekularen Größen wurden mit Hilfe eines vorgefärbten Markers SDS-7B (Sigma) bestimmt.

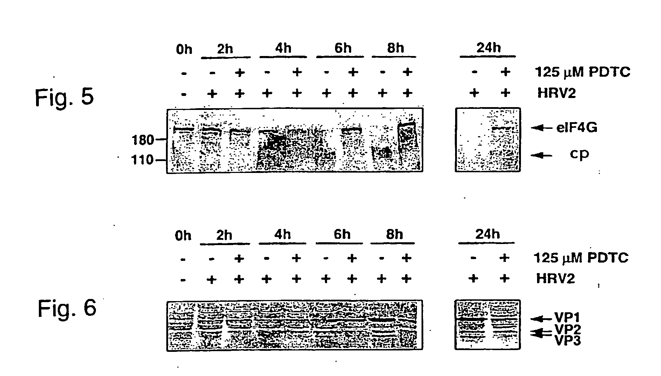

In HRV2-infizierten HeLa-Zellen (100 TCID₅₀ pro Zelle) kann das Spalten von eIF4GI 4 Stunden nach Infektion detektiert werden (cp steht für Spaltprodukt "cleavage product"), die vollständige Spaltung ist 8 Stunden nach der Infektion erreicht (Fig.5). Zu diesen Zeitpunkten ist in infizierten Zellen in Gegenwart von PDTC keine Spaltung des eIF4GI detektierbar. Zu späteren Zeitpunkten, etwa 24 Stunden nach Infektion kann auch in PDTC behandelten Zellen eine leichte eIF4GI-Spaltung detektiert werden.

Dies zeigt, dass entweder die Protease-Funktion blockiert ist oder die Menge an viralen Proteinen stark reduziert ist.

### Beispiel 6: Detektion von viralen Hüllproteinen

Um den Einfluss von PDTC auf die Expression von viralen Proteinen zu testen, wurden Kapsid-Proteine von HRV2 in Protein-Extrakten von HRV2-infizierten HeLa-Zellen (100 TCID₅₀ pro Zelle) durch Western Blot-Analyse detektiert (s. Fig.6). Die Western Blot-Analyse wurde wie oben unter Beispiel 5 beschrieben durchgeführt, wobei ein polyklonales Kaninchen-Antiserum gegen HRV2 verwendet wurde. Signifikante Mengen der rhinoviralen Proteine VP1, VP2 und VP3 wurden in unbehandelten Zellen zu einem Zeitpunkt von 6 Stunden nach Infektion detektiert. Eine PDTC-Behandlung verhindert die Expression dieser Kapsid-Proteine innerhalb der ersten 8 Stunden nach Infektion. Die schwache Expression von VP1, VP2 und vP3 wurde erst zu einem späten Zeitpunkt von etwa 24 Stunden nach Infektion detektiert.

Damit ist gezeigt, dass PDTC zu einer verspäteten Produktion von viralen Proteinen führt, was die anti-rhinovirale Wirkung von PDTC als auch dessen Schutzwirkung auf Zellen erklärt.

### Beispiel 7: Bestimmung der Abhängigkeit der antiviralen Wirkung vom Redox-Potential

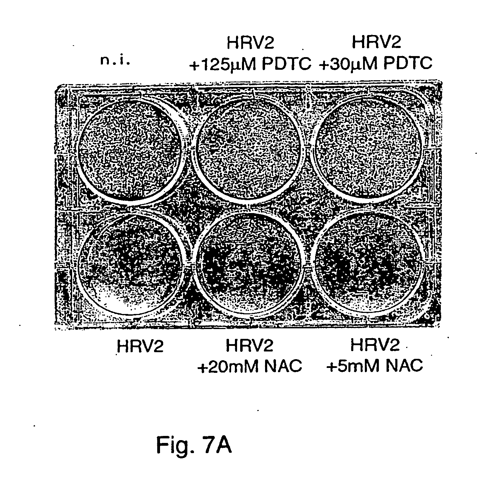

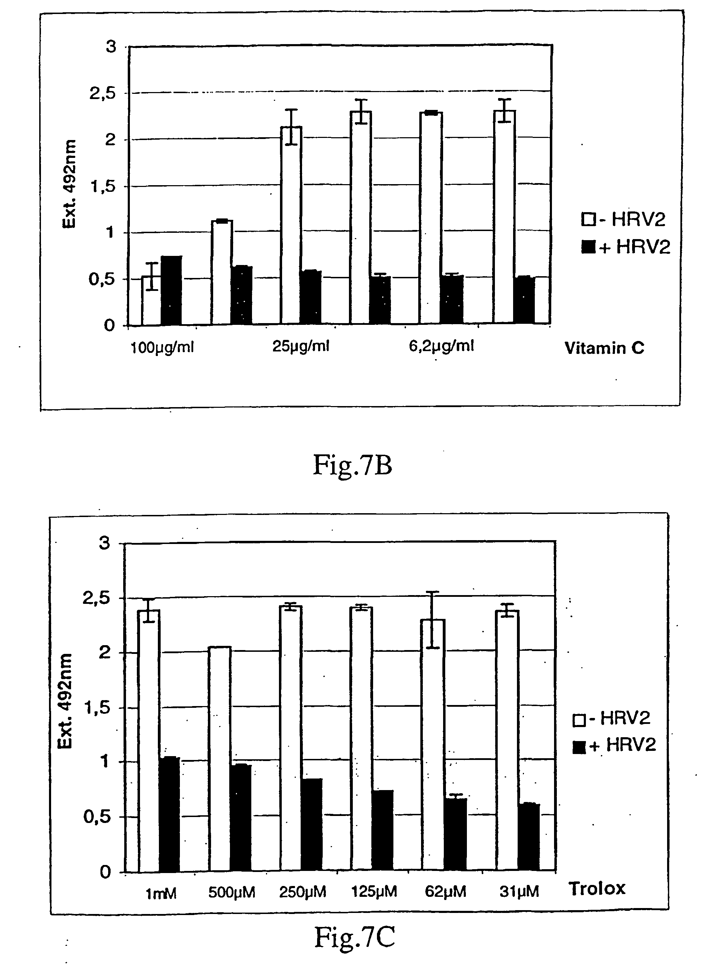

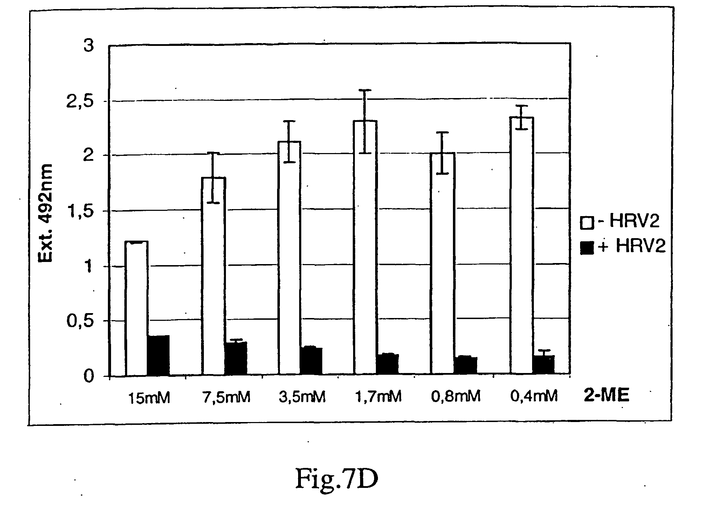

Es wurden weitere Antioxidantien auf ihre Inhibitor-Wirkung während einer HRV2-Infektion von HeLa-Zellen getestet (Fig.7). HeLa-Zellen wurden in einer 6 Well-Platte mit HRV2 (100 TCID₅₀ pro Zelle) infiziert ("n.i." bedeutet nicht infiziert). 1h nach der Infektion wurde das Medium entfernt und frisches Medium bzw. Medium mit PDTC oder NAC in verschiedenen Konzentrationen zugegeben. Nach 24 h wurde der Zellrasen mit PBS gewaschen und mit Kristallviolett gefärbt. In Fig.7A ist ersichtlich, dass die Infektion mit HRV2 den zellrasen zerstört, PDTC kann diesen Effekt verhindern, nicht aber NAC. Die Wirkungsweise von Vitamin C, Trolox und β-Mercaptoethanol (2-ME) wurde wie in Beispiel 3 beschrieben bestimmt (Fig.7 B,C,D). Es konnte überraschenderweise gezeigt werden, dass all diese antioxidativen Substanzen keinerlei Schutzwirkung während viraler Vermehrung hatten. Als Kontrolle wurden die toxischen Effekte der Substanzen in Abwesenheit von virus getestet. Eine hohe Dosis von Vitamin C (100 µg/ml) inhibierte das zellenwachstum stark.

Damit wurde gezeigt, dass die antivirale Wirkung von PDTC nicht auf dessen antioxidative Wirkung zurückzuführen ist, sondern offensichtlich mit anderen Eigenschaften zusammenhängt.

### Beispiel 8: Wirkung von PDTC auf die Influenza-Virus-Replikation

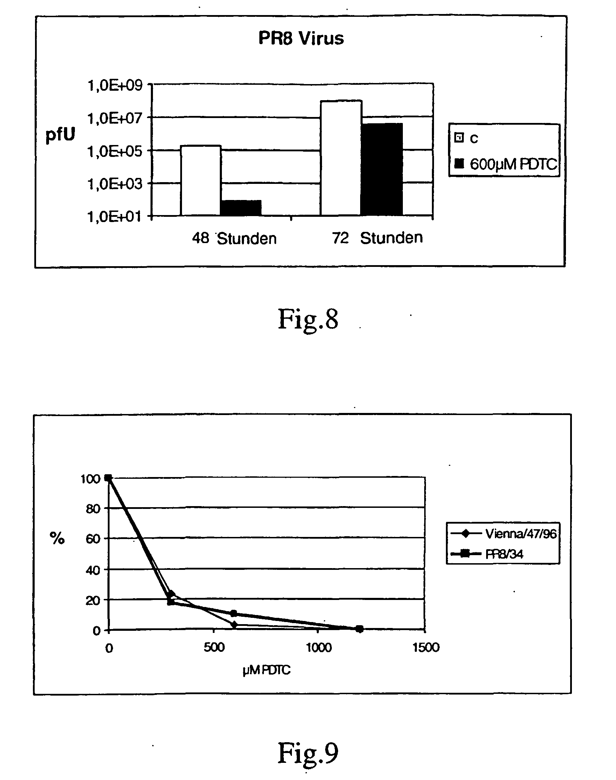

5x10⁵-Verozellen wurden mit Influenzavirus A/PR8/34 oder Vienna/47/96 mit einer m.o.i. (multiplicity of infection) von 0,01 infiziert und 1 h bei Raumtemperatur inkubiert. Anschließend wurde das Inokulum entfernt und Infektionsmedium mit 5 µg/ml Trypsin und 600 µM PDTC zugegeben. Der Überstand wurde nach 48 h und 72 h entfernt und der Virustiter im Überstand mittels einem Standard-Plattenassay gemessen. Wie in Fig.8 gezeigt, wurde der Virustiter von A/PR8/34 in Gegenwart von PDTC um mehr als 2 log Stufen verringert gegenüber der Kontrollprobe (c).

Um die Wirksamkeit des PDTC zu testen wurde ein TCID₅₀ (50% tissue culture infective dose)-Assay mit und ohne PDTC durchgeführt. TCID₅₀ wurde gemäß der Methode von Kaerber bei jeder Konzentration berechnet: 96 Well-Mikrotiterplatten wurden mit 2fachen Verdünnungsreihen des angegebenen Virus infiziert, 1h nach der Infektion wurde der Überstand entfernt und Medium umfassend die angegebenen Konzentrationen von PDTC zugegeben. Die Anzahl der infizierten Wells wurde nach 4 Tagen bestimmt. In Fig.9 ist gezeigt, dass bei einer Konzentration von 300 µM PDTC der TCID₅₀ um 76,4% bzw. 82,2% für A/PR8/34 bzw. A/Vienna/47/96 reduziert war. Eine Inhibierung von über 99,9% wurde für beide Viren bei einer Konzentration von 1200 µM erreicht.

### Beispiel 9: Bestimmung der wirksamen Konzentration von PDTC

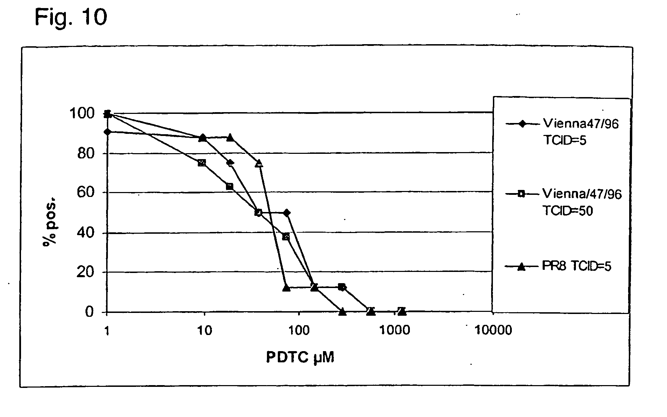

Mittels eines CPE (cytopathic effect) Reduktions-Assays wurde die wirksame Konzentration von PDTC bestimmt: Verozellen wurden in 96 Well-Mikrotiterplatten kultiviert und mit 5 TCID₅₀ pro Well und 50 TCID₅₀ pro Well Influenza A/PR8/34 sowie 5 TCID₅₀ pro Well Influenza A/Vienna/47/96 infiziert. 1 h nach der Infektion wurde der Überstand entfernt und Medium mit 5 µg/ml Trypsin und einer 2fachen Verdünnungsreihe von PDTC beginnend bei einer Konzentration von 1200 µM zugegeben. Die Platten wurden während der darauffolgenden 4 Tage visuell auf cytopathische Effekte untersucht. Das Auftreten von cytopathischen Effekten im Verhältnis zur Kontrollprobe wurde für jede Konzentration berechnet. 100 % positiv bedeutet die komplette Lyse in allen Wells. In Fig. 10 ist ersichtlich, dass eine 50% Reduktion von positiven Wells bei Konzentrationen zwischen 50 und 100 µM PDTC erreicht wurde, eine komplette Hemmung des cytopathischen Effekts wurde bei einer Konzentration von 600 µM PDTC für alle Viren erreicht.

### Beispiel 10: Bestimmung der PDTC Wirkung in vivo

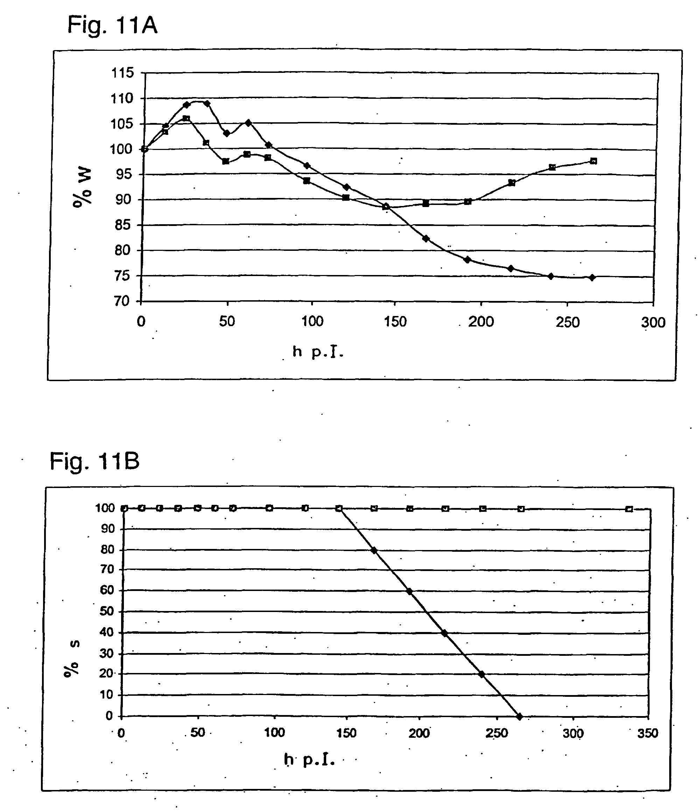

10 C57/BL6-Mäuse wurden intranasal mit einer letalen Dosis (50 µl 10⁷ pfU) des A/PR8/34-Virus inokuliert. 1 h danach wurden sie intranasal mit 25 µl 600 mM PDTC bzw. 25 µl PBS behandelt. Die Mäuse wurden während der ersten 48 h alle 12 h, anschließend alle 24 h untersucht und behandelt. Das Gewicht der Mäuse wurde gemessen und in % des ursprünglichen Gewichts angegeben (% w)(Fig. 11A). In Fig.11B ist gezeigt, dass alle mit PDTC behandelten Mäuse (Quadrat) die Virusinfektion überlebten und 7 Tage nach Infektion Gewicht zunahmen. Alle mit PBS behandelten Mäuse (Rauten) starben innerhalb von 12 Tagen nach der Infektion (% s = % Überlebende).

Damit ist gezeigt, dass PDTC alleine eine starke Wirksamkeit gegen Influenza Virus Infektionen in vitro und in vivo aufweist.

### Beispiel 11: Antivirale Aktivität von PDTC gegen Equines Rhinitis A-Virus (ERAV)

Die Reduktion der viralen Multiplikation von ERAV durch Zugabe von PDTC wurde wie folgt untersucht: Vero-Zellen wurden mit 10 TCID₅₀ pro Zelle ERAV infiziert. Gleichzeitig wurden unterschiedliche Konzentrationen PDTC zugegeben (1 mM - 50 µm). 4 h nach der Infektion wurde das Inokulum entfernt und frisches Medium mit PDTC zugegeben. 24 h nach der Infektion wurden die Überstände entnommen und der virale Titer in einem Standard-Plattenassay bestimmt .

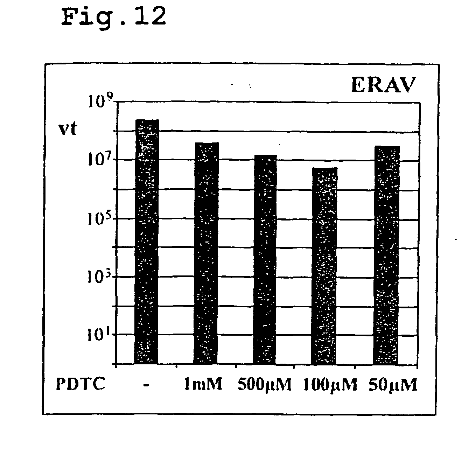

Fig.12 zeigt, dass der Virustiter (vt) im Überstand im Vergleich zu unbehandelten Zellen (-) abnimmt.

### Beispiel 12: Wirkung von PDTC auf die Vermehrung von Maul- und Klauen-Seuche-Virus (MKS) in Zellkultur

Mittels eines CPE ("cytopathic effect") Reduktions-Assays wurde die wirksame Konzentration von PDTC auf die durch MKS-Virus bedingte Zellzerstörurig ermittelt.

IB-RS-2-Zellen wurden in 96 Well-Mikrotiterplatten kultiviert und mit MKS-Virus O-Manisa mit 0,1 TCID₅₀ pro Zelle infiziert und 1h bei 37°C inkubiert. Anschließend wurde das Inokulum entfernt und Infektionsmedium mit den angegebenen Konzentrationen von PDTC (10 µM bis 1200 µM) zugegeben. Die Anzahl der infizierten Wells wurde durch mikroskopische Beobachtung des cytopathischen Effekts nach 24 h bestimmt.

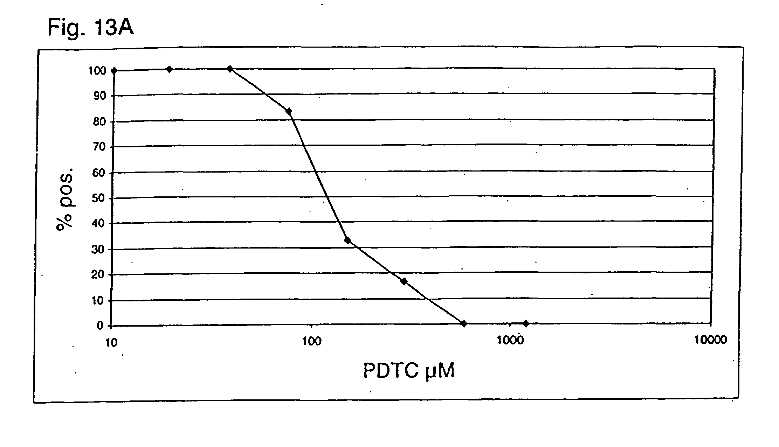

In Fig.13A ist gezeigt, dass die Anzahl der infizierten Wells (% pos.) nach 24 h von der PDTC-Konzentration abhängig ist. 600 µM PDTC schützen die Zellen zu 100% vor virusbedingtem cytopathischen Effekt. Eine 50% Reduktion von infizierten Wells wird im Konzentrationsbereich zwischen 75 µM und 150 µM erreicht.

Um die Wirkung von PDTC auf die MKS-Virus-Replikation zu testen, wurden IB-RS-2-Zellen in T25cm² Zellkultur-Flaschen mit MKS-Virus O-Manisa mit 0,001 TCID₅₀ pro Zelle infiziert und 1h bei 37°C inkubiert. Anschließend wurde das Inokulum entfernt und Infektionsmedium mit den angegebenen Konzentrationen PDTC (0 µM bis 200 µM) zugegeben. 24 h nach der Infektion wurden die Überstände entnommen und der virale Titer (TCID) in einem Standard-Plattenassay bestimmt .

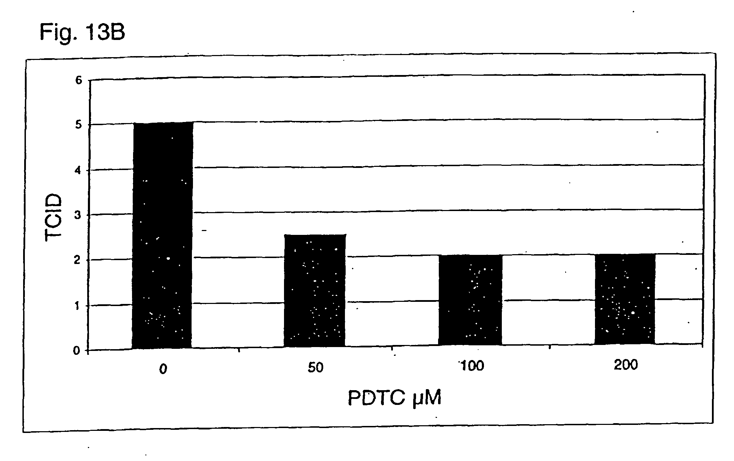

Wie in Fig.13B gezeigt, wird der Virustiter von MKS-Virus O-Manisa in Gegenwart von PDTC im Vergleich mit der Kontrollprobe (0) um mehr als 2 log Stufen verringert. Der Kontrollprobe wurde kein PDTC zugesetzt.

### Beispiel 13: Wirkung von PDTC auf die Vermehrung von FSME in Zellkultur

FSME-Viren gehören weder zu den Picornaviren noch lösen sie eine Erkrankung im respiratorischen Trakt aus.

Konfluente Monolayers von BHK-21-Zellen (ATCC) wurden mit 10 pfu/Zelle FSME-Virus (Neudörfel) in Gegenwart von folgenden Konzentrationen PDTC infiziert: 1000 µM, 500 µM, 250 µM, 125 µM, 62,5 µM, 31,25 µM, 15,6 µM, 7, 8 µM, 3, 9 µM, 1,95 µM, 0,975 µM.

Anschließend wurden die Zellen 4 Tage bei 37°C inkubiert und der Zellmonolayer mikroskopisch analysiert. Die Virusvermehrung wurde durch Verwendung eines Enzymimmunoassays bestimmt. Die mikroskopische Analyse zeigt keine Hinweise auf toxische Effekte von PDTC. Eine Quantifizierung der Virusvermehrung zeigt, dass PDTC in keiner der getesteten Konzentrationen in der Lage ist, die Virusvermehrung zu reduzieren.

In Analogie wurde untersucht, ob PDTC in Zellen in Suspension die Virusvermehrung beeinflussen kann: Es wurden dieselben Konzentrationen wie oben getestet. Auch in diesem Versuch kann kein Hinweis auf eine Reduzierung der Virusvermehrung durch PCTC gefunden werden.

### Beispiel 14: Wirkung von PDTC auf EMC-infizierte Mäuse

C57B16-Mäuse wurden mit 10 TCID50 EMC-virus intraperitoneal infiziert. Die Kontrollgruppe wurde anschließend ein Mal täglich mit 50 µl PBS intraperitoneal behandelt. Zwei weitere Gruppen wurden mit 50 µl 50 mM PDTC pro Tag behandelt, wobei bei einer Gruppe die Behandlung gleichzeitig mit der Infektion begonnen wurde (PDTC), bei der anderen Gruppe erst 24 h nach der Infektion (PDTC 24hpi).

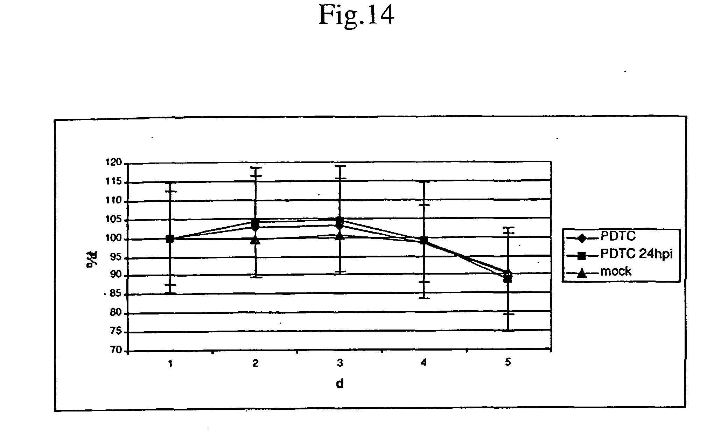

In Figur 14 ist die Veränderung des Mausgewichts nach Beginn der Infektion gezeigt. Es ist klar ersichtlich, dass sich behandelte und unbehandelte Tiere nicht unterscheiden. Im Mittel tritt in den infizierten Mäusen der Tod in allen Gruppen in 5,5 Tagen ein.

Damit ist gezeigt, dass PDTC keine Wirkung auf eine EMC-Infektion, das zwar zu den Picornaviren zählt, jedoch keine Erkrankung im respiratorischen Trakt sondern in Nervenzellen auslöst, hat.

## Patentansprüche

1. Verwendung von Dithiocarbamat-Verbindungen der Strukturformel R₁R₂NCS₂H, worin R₁ und R₂ unabhängig voneinander ein gerades oder verzweigtes C₁-C₃-Alkyl darstellen oder mit dem Stickstoffatom einen aliphatischen Ring mit 4 bis 6 C-Atomen bilden, wobei R₁, R₂ oder der aliphatische Ring gegebenenfalls mit einem oder mehreren Substituenten ausgewählt aus OH, NO₂, NH₂, COOH, SH, F, Cl, Br, I, Methyl oder Ethyl substituiert ist, und oxidierte Formen dieser Verbindungen, insbesondere Dimere hievon, sowie pharmazeutisch akzeptable Salze hievon, zur Herstellung eines Mittels zur Behandlung oder Verhinderung einer Infektion durch RNA-viren, die den respiratorischen Trakt bei Mensch und Säugetieren befallen und dort eine Erkrankung auslösen.

2. Verwendung nach Anspruch 1, **dadurch gekennzeichnet, dass** R₁ und R₂ unabhängig voneinander ein C₁-C₃-Alkyl darstellen oder mit dem Stickstoffatom einen aliphatischen Ring mit 4 bis 6 C-Atomen bilden.

3. Verwendung nach Anspruch 1 oder 2, **dadurch gekennzeichnet, dass** die Dithiocarbamat-Verbindung ausgewählt ist aus Pyrrolidin-Dithiocarbamat und N,N-Diethyl-Dithiocarbamat.

4. Verwendung nach einem der Ansprüche 1 bis 3, **dadurch gekennzeichnet, dass** die Virus-Infektion eine Infektion mit Picornavirus, Orthomyxo- oder Paramyxovirus ist.

5. Verwendung nach Anspruch 4, **dadurch gekennzeichnet, dass** das orthomyxovirus ein humanes Influenzavirus ist, insbesondere ausgewählt aus der Gruppe bestehend aus Influenza A, Influenza B, Influenza C bzw. das Paramyxovirus ein Parainfluenzavirus oder Pneumovirus ist.

6. verwendung nach Anspruch 4, **dadurch gekennzeichnet, dass** das orthomyxovirus ein Säugetier-Influenza A-Virus ist.

7. verwendung nach einem der Ansprüche 1 bis 6, **dadurch gekennzeichnet, dass** das Picornavirus ein Rhinovirus, insbesondere humanes oder Pferde-Rhinovirus, ein Enterovirus, insbesondere Ente rovirus 70, 71 oder coxsackievirus, bzw. ein Aphthovirus, insbesondere das Maul- und Klauenseuche-Virus oder equines Rhinitisvirus A, ist.

8. Verwendung nach einem der Ansprüche 1 bis 7, **dadurch gekennzeichnet, dass** die Pithiocarbamat-Verbindung in dem Mittel in einer Konzentration von 0,01 bis 5000 mM, vorzugsweise 1 bis 300 mM, besonders bevorzugt 10 bis 100 mM, vorgesehen ist.

9. Verwendung nach einem der Ansprüche 1 bis 7, **dadurch gekennzeichnet, dass** die Dithiocarbamat-verbindungen in dem Mittel in einer Konzentration von 10 mM bis 1 M vorgesehen ist.

10. Verwendung nach einem der Ansprüche 1 bis 9, **dadurch gekennzeichnet, dass** das Mittel weiters einen pharmazeutisch akzeptablen Träger umfasst.

11. Verwendung nach einem der Ansprüche 1 bis 10, **dadurch gekennzeichnet, dass** das Mittel ein oral, intranasal, intravenös, rektal, parenteral oder als Augen- oder ohrentropfen, als Gurgellösung oder Aerosol zu verabreichendes Mittel ist.

12. Verwendung nach einem der Ansprüche 1 bis 11, **dadurch gekennzeichnet, dass** das Mittel weitere antivirale Substanzen umfasst.

13. Verwendung nach einem der Ansprüche 1 bis 12, **dadurch gekennzeichnet, dass** das Mittel eine Kombination von zumindest zwei verschiedenen Dithiocarbamat-Verbindungen umfasst.

14. Verwendung nach einem der Ansprüche 1 bis 13, **dadurch gekennzeichnet, dass** das Mittel weitere Substanzen, ausgewählt aus Antibiotika, Impfstoffen, Immunsuppressiva, Stabilisatoren, immunstimulierenden Substanzen, Blutprodukten oder Mischungen davon, umfasst.

15. Verwendung nach einem der Ansprüche 1 bis 14, **dadurch gekennzeichnet, dass** das Mittel zur Inhibierung der virusvermehrung eingesetzt wird.

## Claims

1. Use of dithiocarbamate compounds having the structural formula R₁R₂NCS₂H, in which R₁ and R₂, independently of each other, represent a straight or branched C₁ - C₄ alkyl, or, with the nitrogen atom, form an aliphatic ring with 4 to 6 C atoms, in which R₁, R₂, or the aliphatic ring is optionally substituted with one or more substituents selected from OH, NO₂, NH₂, COOH, SH, F, Cl, Br, I, methyl or ethyl, and oxidized forms of these compounds, in particular dimers thereof, as well as pharmaceutically acceptable salts thereof, to prepare an agent for treating or preventing an infection by RNA viruses which attack the respiratory tract in humans and mammals and cause disease there.

2. Use according to claim 1, **characterized in that** R₁ and R₂, independently of each other, represent a C₁ - C₃ alkyl, or, with the nitrogen atom, form an aliphatic ring with 4 to 6 C atoms.

3. Use according to claim 1 or 2, **characterized in that** the dithiocarbamate compound is selected from pyrrolidine dithiocarbamate and N,N-diethyl dithiocarbamate.

4. Use according to any one of claims 1 to 3, **characterized in that** the virus infection is an infection with picornavirus, orthomyxovirus or paramyxovirus.

5. Use according to claim 4, **characterized in that** the orthomyxovirus is a human influenza virus, in particular selected from the group consisting of influenza A, influenza B, influenza C, or the paramyxovirus is a parainfluenza virus or pneumovirus, respectively.

6. Use according to claim 4, **characterized in that** the orthomyxovirus is a mammalian influenza A virus.

7. Use according to any one of claims 1 to 6, **characterized in that** the picornavirus is a rhinovirus, in particular human or equine rhinovirus, an enterovirus, in particular enterovirus 70, 71, or Coxsackie virus, or an aphthovirus, respectively, in particular the foot and mouth disease virus or equine rhinitis virus A.

8. Use according to any one of claims 1 to 7, **characterized in that** the dithiocarbamate compound is provided in the agent at a concentration of from 0.01 to 5000 mM, preferably 1 to 300 mM, and especially preferably 10 to 100 mM.

9. Use according to any one of claims 1 to 7, **characterized in that** the dithiocarbamate compound is provided in the agent at a concentration of 10 mM to 1 M.

10. Use according to any one of claims 1 to 9, **characterized in that** the agent further comprises a pharmaceutically acceptable carrier.

11. Use according to any one of claims 1 to 10, **characterized in that** the agent is an agent to be administered orally, intranasally, intravenously, rectally, parenterally or as eye or ear drops, as a gargle, or an aerosol.

12. Use according to any one of claims 1 to 11, **characterized in that** the agent comprises further antiviral substances.

13. Use according to any one of claims 1 to 12, **characterized in that** the agent comprises a combination of at least two different dithiocarbamate compounds.

14. Use according to any one of claims 1 to 13, **characterized in that** the agent comprises further substances selected from antibiotics, vaccines, immune suppressants, stabilizers, immune-stimulating substances, blood products, or mixtures thereof.

15. Use according to any one of claims 1 to 14, **characterized in that** the agent is used to inhibit virus propagation.

## Revendications

1. Utilisation de composés dithiocarbamates de formule développée R₁R₂NCS₂H dans lesquels R₁ et R₂ représentent indépendamment l'un de l'autre des alkyles C₁-C₄ linéaires ou ramifiés ou forment, avec l'atome d'azote, un cycle aliphatique de 4 à 6 atomes de carbone, où R₁, R₂ ou le cycle aliphatique est substitué, le cas échéant, par un ou plusieurs substituants sélectionnés parmi OH, NO₂, NH₂, COOH, SH, F, Cl, Br, I, le méthyle ou l'éthyle et les formes oxydées de ces composés, en particulier leurs dimères ainsi que leurs sels pharmaceutiquement acceptables, pour le fabrication d'un médicament traitant ou prévenant une infection par les virus à ARN qui affectent les voies respiratoires des humains et des mammifères et y provoquent une maladie.

2. Utilisation selon la revendication 1, **caractérisée en ce que** R₁ et R₂ représentent indépendamment l'un de l'autre des alkyles C₁-C₃ ou forment, avec l'atome d'azote, un cycle aliphatique de 4 à 6 atomes de carbone.

3. Utilisation selon les revendications 1 ou 2, **caractérisée en ce que** le composé de dithiocarbamate est choisi parmi le pyrrolidine dithiocarbamate et le N,N-diéthyle dithiocarbamate.

4. Utilisation selon une des revendications 1 à 3, **caractérisée en ce que** l'infection virale est une infection à picomavirus, à orthomyxovirus ou à paramyxovirus.

5. Utilisation selon la revendication 4, **caractérisée en ce que** l'orthomyxovirus est un virus humain d'influenza, en particulier du groupe constitué par l'influenza de type A, l'influenza de type B, l'influenza de type C ou le paramyxovirus, un virus de parainfluenza ou un pneumovirus.

6. Utilisation selon la revendication 4, **caractérisée en ce que** l'orthomyxovirus est un virus d'influenza de type A de mammifère.

7. Utilisation selon une des revendications 1 à 6, **caractérisée en ce que** le picomavirus est un rhinovirus, en particulier un rhinovirus humain ou équin, un entérovirus, en particulier un entérovirus 70, 71 ou un coxsackievirus ou un aphtovirus, en particulier le virus de la fièvre aphteuse ou le virus de la rhinite équine A.

8. Utilisation selon une des revendications 1 à 7, **caractérisée en ce que** la concentration du composé de dithiocarbamate dans le médicament est prévue de 0,01 à 5000 mM, de préférence de 1 à 300 mM, particulièrement de préférence de 10 à 100 mM.

9. Utilisation selon une des revendications 1 à 7, **caractérisée en ce que** la concentration des composés dithiocarbamates dans le médicament est prévue de 10 mM à 1 M.

10. Utilisation selon des revendications 1 à 9, **caractérisée en ce que** le médicament comprend, en plus, un support pharmaceutiquement acceptable.

11. Utilisation selon une des revendications 1 à 10, **caractérisée en ce que** le médicament est administrable par voies orale, intranasale, intraveineuse, rectale, parentérale ou sous la forme de gouttes oculaires ou auriculaires; de solution pour gargarisme ou d'aérosol.

12. Utilisation selon une des revendications 1 à 11, **caractérisée en ce que** le médicament inclut d'autres substances anti-virales.

13. Utilisation selon une des revendications 1 à 12, **caractérisée en ce que** le médicament englobe une combinaison d'au moins deux composés de dithiocarbamate différents.

14. Utilisation selon une des revendications 1 à 13, **caractérisée en ce que** le médicament inclut d'autres substances, choisies parmi les antibiotiques, vaccins, immunosuppresseurs, stabilisateurs, substances immunostimulantes, produits sanguins ou mélanges de ces différents produits.

15. Utilisation selon une des revendications 1 à 14, **caractérisée en ce que** le médicament est utilisé pour inhiber la multiplication virale.
